# FASCICULE DE BREVET EUROPEEN

(11) **EP 1 572 695 B1**
(45) Date de publication et mention de la délivrance du brevet: **17.12.2008**
(21) Numéro de dépôt: 03799628.7
(22) Date de dépôt: 18.12.2003
(51) Int. Cl.: C07D 487/22, A61K 51/00, A61K 49/00, A61K 31/409, A61P 35/00

(54) **DERIVES DES PORPHYRINES, LEURS PROCEDES D' OBTENTION, ET LEURS UTILISATIONS EN RADIOIMMUNOTHERAPIE**
PORPHYRIN-DERIVATE, VERFAHREN ZU IHRER HERSTELLUNG UND IHRE VERWENDUNG IN RADIOIMMUNOTHERAPIE
PORPHYRIN DERIVATIVES, METHODS FOR OBTAINING SAME, AND USE THEREOF IN RADIOIMMUNOTHERAPY

(30) Priorité: 20.12.2002 FR 0216371; 22.10.2003 FR 0312341
(43) Date de publication de la demande: 14.09.2005
(73) Titulaire: CENTRE NATIONAL DE LA RECHERCHE SCIENTIFIQUE, 75794 Paris Cedex 16 (FR); UNIVERSITE DE RENNES I, F-35065 Rennes (FR)
(72) Inventeur: BOITREL, Bernard, Philippe, Albert, F-35520 Melesse (FR)
(74) Mandataire: Grosset-Fournier, Chantal Catherine
(86) Numéro de dépôt international: PCT/FR2003/003794
(87) Numéro de publication internationale: WO 2004/063199

(56) Documents cités:
- WO-A-94/19352
- FR-A- 2 709 491
- DIDIER ET AL: "A Versatile and Convenient Method for the Functionalization of Porphyrins" EUROPEAN JOURNAL OF ORGANIC CHEMISTRY, WILEY-VCH VERLAG, WEINHEIM, DE, 2001, pages 1917-1926, XP002247504 ISSN: 1434-193X cité dans la demande
- BOITREL ET AL: "Synthesis and Spectral and Structural Characterization of a New Series of Bis-Strapped Chiral Porphyrins Derived from L-Proline" EUROPEAN JOURNAL OF ORGANIC CHEMISTRY, WILEY-VCH VERLAG, WEINHEIM, DE, 2001, pages 4213-4221, XP002247505 ISSN: 1434-193X
- BOITREL ET AL: "Investigation of the Enantioselectivity Observed in Epoxidation Reactions atalysed by Bis-Strapped Chiral Porphyrins Derived from L-Proline" EUROPEAN JOURNAL OF INORGANIC CHEMISTRY, WILEY-VCH VERLAG, WEINHEIM, DE, 2002, pages 1666-1672, XP002247506 ISSN: 1434-1948
- MICHAUDET L ET AL: "Synthesis and crystal structure of an unprecedented bismuth porphyrin containing ester pendant arms" CHEMICAL COMMUNICATIONS - CHEMCOM, ROYAL SOCIETY OF CHEMISTRY, GB, 2000, pages 1589-1590, XP002247507 ISSN: 1359-7345
- MOMENTEAU M ET AL: "KINETIC EVIDENCE FOR DIOXYGEN STABLIZATION IN OXYGENATED IRON(II)-PORPHYRINS BY DISTAL POLAR INTERACTIONS" JOURNAL OF THE CHEMICAL SOCIETY, CHEMICAL COMMUNICATIONS, CHEMICAL SOCIETY. LETCHWORTH, GB, 1982, pages 341-343, XP009013743 ISSN: 0022-4936
- MOMENTEAU M ET AL: "SYNTHESIS AND CHARACTERIZATION OF A NEW SERIES OF IRON(II) SINGLE-FACE HINDERED PORPHYRINS. INFLUENCE OF CENTRAL STERIC HINDRANCE UPON CARBON MONOXIDE AND OXYGEN BINDING" JOURNAL OF THE CHEMICAL SOCIETY, PERKIN TRANSACTIONS 2, CHEMICAL SOCIETY. LETCHWORTH, GB, 1987, pages 249-257, XP009013750 ISSN: 1472-779X
- BHALGAT M K ET AL: "Preparation and Biodistribution of Copper-67-Labeled Porphyrins and Porphyrin-A6H Immunoconjugates" NUCLEAR MEDICINE AND BIOLOGY, ELSEVIER SCIENCE PUBLISHERS, NEW YORK, NY, US, vol. 24, no. 2, 1 février 1997 (1997-02-01), pages 179-185, XP004058589 ISSN: 0969-8051
- KURODA, Y. ET AL.: "Chiral Amino Acid Recognition by a Porphyrin-Based Artificial Receptor" J.AM.CHEM.SOC., vol. 117, 1995, pages 10950-10958, XP002276870

## Description

La présente invention a pour objet de nouveaux dérivés des porphyrines, leurs procédés d'obtention, et leurs utilisations en radiothérapie ou radioimmunothérapie.

Les traitements actuellement administrés dans la lutte contre le cancer concernent principalement des drogues chimiques, et l'utilisation de sources de radiation. Le principal problème engendré par ce type de traitement est la non spécificité de ces techniques thérapeutiques, ce qui entraîne, par conséquent, l'endommagement, sans distinction aucune, des cellules saines.

La découverte des anticorps monoclonaux dans les années 70 a apporté un grand espoir dans les domaines du diagnostique et de la thérapie des cancers. Cette technique nouvelle paraît être, en effet, une solution aux problèmes de non spécificité des agents antitumoraux. Cependant, les anticorps monoclonaux capables' de reconnaître les antigènes à la surface des tumeurs ne présentent pas une toxicité suffisante pour la détruire. Par contre, en associant cette protéine à un élément capable d'éliminer les cellules malades, on forme une entité très intéressante puisque très spécifique et active. Ainsi, la radio immunothérapie associe les propriétés des anticorps monoclonaux et celle des métaux radioactifs. L'anticorps est modifié lors d'un couplage avec un ligand stabilisant le radioélément ou directement avec le radioélément *(*Yuanfang, L.; Chuanchu, W. Pure Appl. Chem. 1991, 63, 427*)*.

De nombreux radioéléments ont déjà fait l'objet d'étude (*Yuanfang, L.;Chuanchu*, *W., susmentionné*) très poussée dans ce domaine. Le bismuth-212 ou-213 est un émetteur α, c'est à dire capable de délivrer une énergie très grande sur une très courte distance, ce qui rend ce métal très attractif pour le traitement de petites cellules tumorales. L'enjeu est donc de taille puisque actuellement très peu d'émetteurs α possèdent un cahier des charges intéressant pour une utilisation éventuelle en radioimmunothérapie (Wibur, D.S. Antibody, Immunoconj. Radiopharm.- 1991, 4, 85*;* Feinendegen, L.; McClure, J; Rad. Res. 1997, 148, 195).

Les premières études concernant le couplage d'un complexe de bismuth avec un anticorps monoclonal et son comportement in vitro, ont été réalisées en 1986 par l'équipe de Kozak (Kozak, R.; Atcher, R.; Gansow, O.; Friedman, A.; Hines, J.; Waldmann, T; Proc. Natl. Acad. Sci. USA 1986, 83).

Ces premières investigations très encourageantes sont réalisées avec l'anhydride isobutylcarbonique du DTPA comme agent complexant, dont la formule est indiquée ci-dessous.

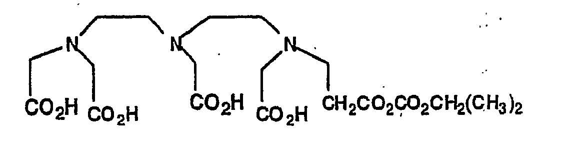

Par la suite, d'autres types de ligands ont été synthétisés afin de parfaire la sphère de coordination du métal et d'induire une plus grande stabilité des complexes formés. Des exemples sont illustrés ci-après avec le DOTA et le cyclohexylbenzyl DTPA (cyDTPA).

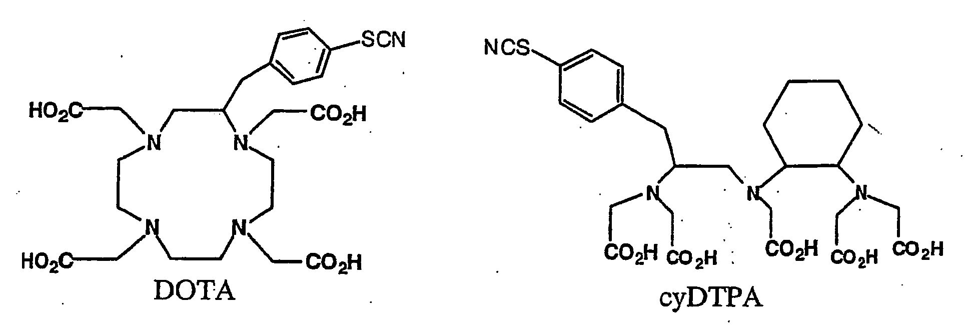

Le cyDTPA, représenté ci-dessus, est actuellement le ligand le plus prometteur. La métallation de ce ligand est très rapide (Brechbiel, M.; Pippin, C; McMurry, T.; Milenic, D.; Roselli, D.; Colcher,D.; Gansow, O.J. Chem. Soc., chem. Soc. 1991, 1169), et le complexe formé est relativement stable, *in vivo*.

Le choix des porphyrines comme ligand n'est pas anodin puisque des études font état d'une accumulation préférentielle des porphyrines dans les tumeurs (Moan, J.; Berg, K. Photochem. Photobiol. 1992, 55, 931), et de leur caractère biocompatible. De plus, ce macrocycle possède des propriétés uniques de par sa forme en disque et sa relative rigidité.

Des études préliminaires, réalisées par les Inventeurs, sur des porphyrines dites planes, telle l'octaéthylporphyrine, ont montré que le métal se situait au dessus du plan de la porphyrine. Le contre-anion a également son importance puisque, dans les complexes isolés, le métal est lié à des anions triflate et nitrate (contre-anion oxygéné). Les Inventeurs ont également tenté de métaller la tétraphénylporphyrine par différents sels de bismuth et notamment le chlorure de bismuth, lorsque la réaction est effectuée sous argon, et suivie par spectroscopie UV-visible, on note un début de métallation mais la majeure partie du ligand de départ n'est pas consommée, et le complexe obtenu n'est pas stable.

La présente invention a pour but de fournir de nouveaux composés permettant la complexation de radioéléments tels que les émetteurs α, et plus particulièrement le bismuth, permettant par rapport aux composés de l'art antérieur de former avec les radioéléments susmentionnés des complexes plus stables, par la présence d'anses préorganisées ne modifiant pas la géométrie du noyau tétrapyrrolique ni ses propriétés électroniques.

L'invention a également pour but de fournir de nouvelles compositions pharmaceutiques susceptibles de pouvoir être utilisées en radiothérapie ou radioimmunothérapie.

La présente invention a pour objet les composés répondant à la formule générale (I) suivante : 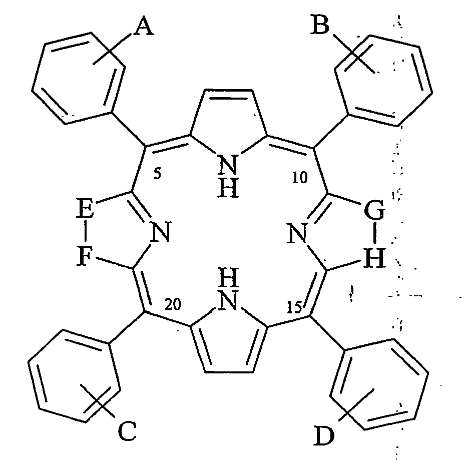 dans laquelle :
- lorsque A forme avec C une chaîne, dite chaîne A-C, de formule (1) suivante :

   -X-Y-C₆H₄-(CH₂)ₙ₁-U-(CH₂)ₙ₂-C₆H₄-Y-X- (1)

   dans laquelle :
   - lorsque X représente NH, O, CO ou CH₂, Y représente respectivement CO, CH₂, NH, ou O,
   - n₁ et n₂, indépendamment l'un de l'autre représente un nombre entier compris entre 1 et 3,
   - U représente un groupe de la forme C(Z,W) ou N(CHRₐ-COOR_{b}), dans lequel
   - Z représente :
      * un groupe électro-attracteur tel que CN, NO₂, ou CO₂⁻,
      * ou un groupe CH₂NR₁R₂, dans lequel R₁ et R₂ représentent, indépendamment l'un de l'autre, H, ou un groupe alkyle linéaire, ramifié, ou cyclique, de 1 à 8 atomes de carbone, ou un groupe aryle ou alkylaryle, ou un anticorps déterminé, le cas échéant lié à la partie CH₂N dudit groupe via un espaceur,
      * ou un groupement aryle substitué par une fonction SO₃R₃, SO₂R₃, p-NO₂ ou o-NO₂, dans laquelle R₃, représente H, ou un cation choisi parmi les métaux alcalins tels que Na⁺, ou K⁺, ou R₃ représente un groupe NR₄R₅ dans lequel R₄ et R₅ représentent, indépendamment l'un de l'autre, un groupe alkyle linéaire, ramifié, ou cyclique, de 1 à 8 atomes de carbone, ou R₃ représente un groupe para-nitro aryle,
   - W représente un groupe CO₂⁻ ou COOR₆ dans lequel R₆ représente H ou un groupe alkyle linéaire, ramifié, ou cyclique, de 1 à 8 atomes de carbone, ou un groupe aryle, ou un alcool appauvri en électrons tel qu'un groupe para-nitro phénol ou ortho-para-nitro phénol,
   - ou Z et W forment en association avec l'atome de carbone qui les portent (indiqué par une flèche ci-après) un cycle désigné acide de Meldrum de formule suivante : 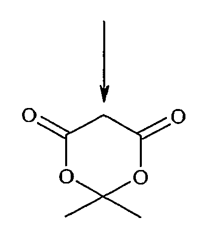
   - Rₐ répond à la définition donnée précédemment pour R₁, ou peut également représenter de préférence la chaîne latérale d'un acide aminé, naturel ou modifié,
   - R_{b} répond à la définition donnée précédemment pour R₁, alors B forme avec D une chaîne, dite chaîne B-D, de formule (1) susmentionnée, lesdites chaînes A-C, et B-D, étant situées indépendamment l'une de l'autre, au dessus (position α) ou au dessous (position β) du plan du macrocyle porphyrinique,
- ou lorsque A forme avec D une chaîne, dite chaîne A-D, de formule (1) susmentionnée, alors B forme avec C une chaîne, dite chaîne B-C, de formule (1) susmentionnée, l'une desdites chaînes A-D ou B-C, étant située au dessus (position α) du plan du macrocyle porphyrinique, tandis que l'autre chaîne A-D ou B-C, est située ou au dessous (position β) du plan du macrocyle porphyrinique,
- E représente en association avec F, et H représente en association avec G, indépendamment les uns des autres, CH=CH, ou CH₂-CH₂.

L'invention a plus particulièrement pour objet les composés de formule (I) susmentionnés, caractérisés en ce que les enchaînements de formule (1) sont choisis parmi les suivants : 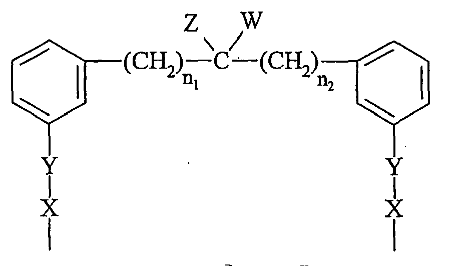 ou 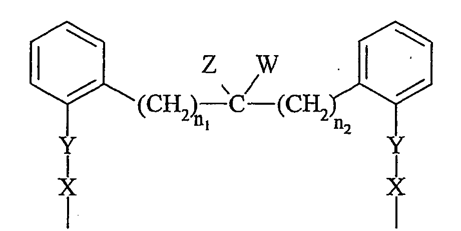 ou 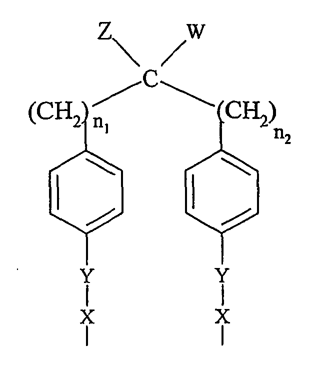 dans lesquels les groupes Z et W sont :
- soit dirigés vers l'intérieur desdits composés et sont situés au dessus ou au dessous du plan du macrocycle porphyrinique suivant que lesdits enchaînements de formule (1) sont situés respectivement en a ou en β, et sont respectivement désignés Ziα et Wiα, ou Ziβ ou Wiβ,
- soit dirigés vers l'extérieur desdits composés, et sont respectivement désignés Ze et We.

L'invention a plus particulièrement encore pour objet les composés tels que définis ci-dessus, caractérisés en ce que A, B, C, et D sont en ortho, ainsi que ceux caractérisés en ce que E représente en association avec F, et H représente en association avec G, CH₂-CH₂.

L'invention concerne plus particulièrement les composés tels que définis ci-dessus, caractérisés en ce que A forme avec C, et B forme avec D, des enchaînements de formule (1) respectivement désignés A-C et B-D, ces deux enchaînements étant situés en a, lesdits composés étant encore désignés composés de formule (Ia).

A ce titre, l'invention a plus particulièrement pour objet les composés susmentionnés de formule (Ia), caractérisés en ce que :
- les enchaînements A-C et B-D comportent chacun un groupe Ziα et un groupe We,
- ou l'enchaînement A-C comporte un groupe Ziα et un groupe We, tandis que l'enchaînement B-D comporte un groupe Ze et un groupe Wiα,
- ou les enchaînements A-C et B-D comportent chacun un groupe Ze et un groupe Wiα.

Des composés de formule (Ia) préférés sont ceux caractérisés par les formules suivantes :

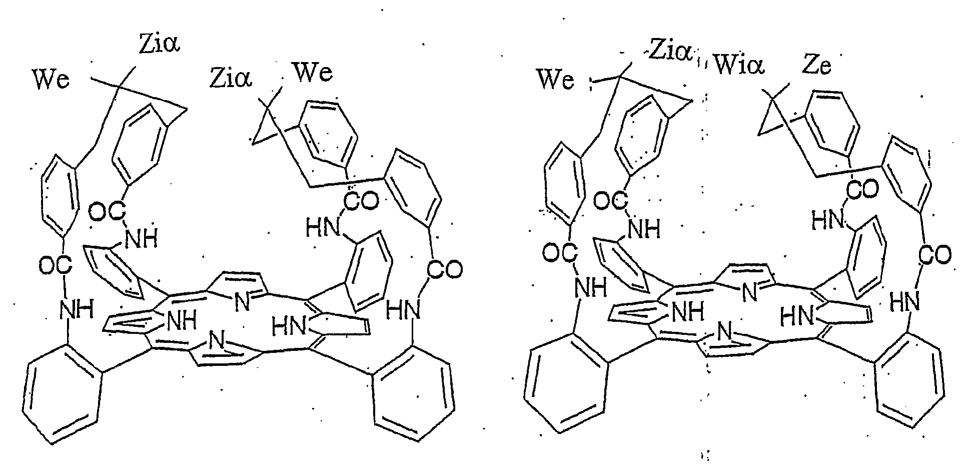

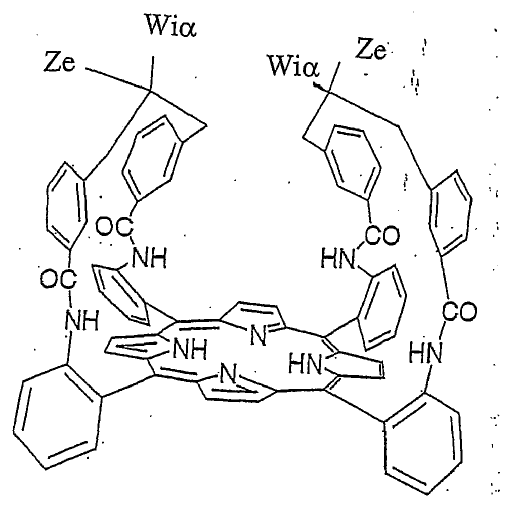

L'invention concerne également les composés de formule (I) tels que définis ci-dessus, caractérisés en ce que A forme avec C un enchaînement A-C de formule (1) situé en position α, et B forme avec D, un enchaînement B-D de formule (1) situé en position β, lesdits composés étant encore désignés composés de formule (Ib).

A ce titre, l'invention a plus particulièrement pour objet les composés susmentionnés de formule (Ib), caractérisés en ce que :
- l'enchaînement A-C comporte un groupe Ziα et un groupe We, tandis que l'enchaînement B-D comporte un groupe Ziβ et un groupe We,
- ou l'enchaînement A-C comporte un groupe Ze et un groupe Wiα, tandis que l'enchaînement B-D comporte un groupe Ziβ et un groupe We,
- ou l'enchaînement A-C comporte un groupe Ze et un groupe Wiα, tandis que l'enchaînement B-D comporte un groupe Ze et un groupe Wiβ.

Des composés de formule (Ib) préférés sont ceux caractérisés par les formules suivantes :

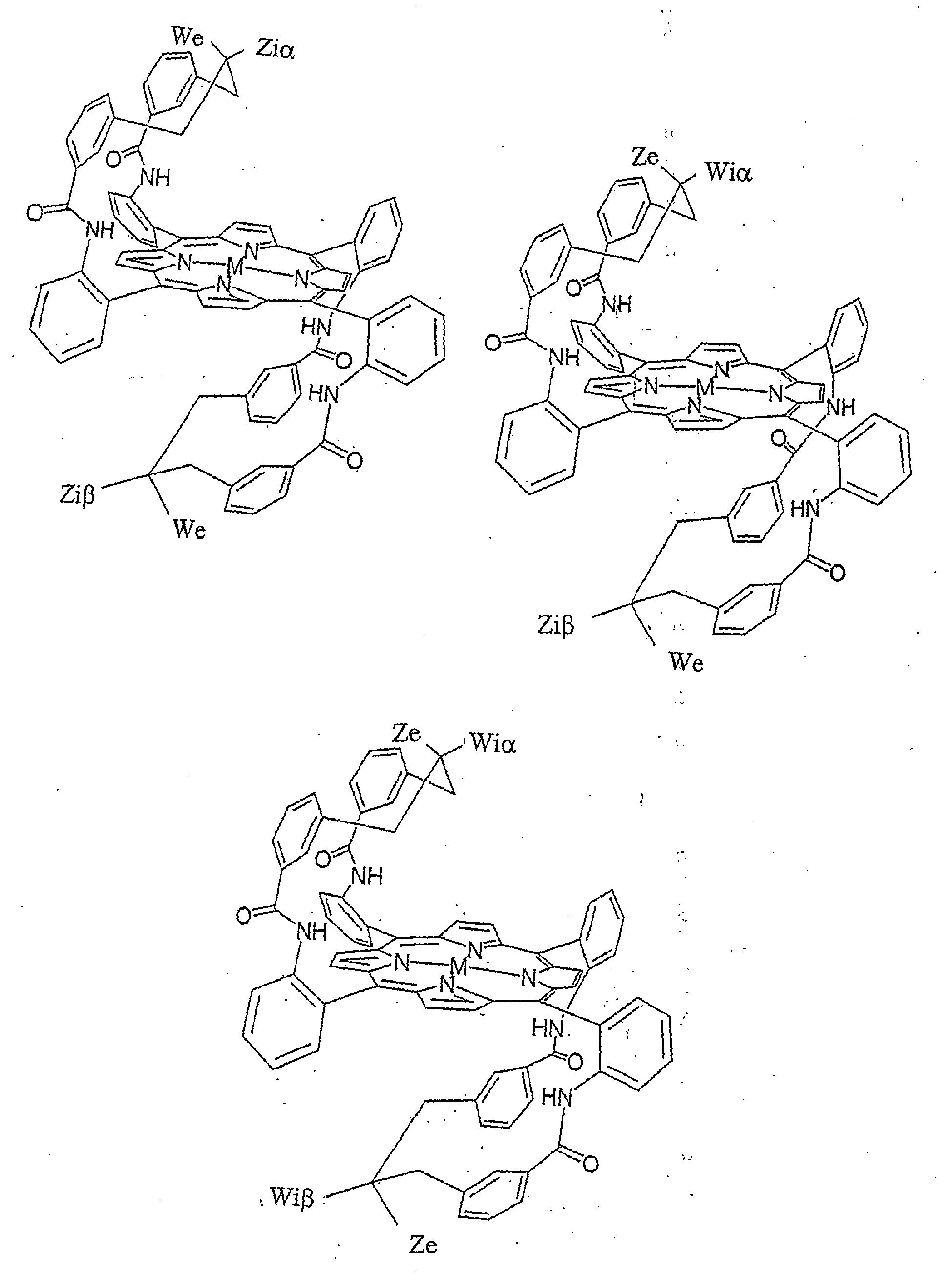

L'invention concerne également les composés de formule (I) tels que définis ci-dessus, caractérisés en ce que A forme avec D un enchaînement À-D de formule (1) situé en position β, et B forme avec C, un enchaînement B-C de formule (1) situé en position α, lesdits composés étant encore désignés composés de formule (Ic).

A ce titre, l'invention a plus particulièrement pour objet les composés susmentionnés de formule (Ic), caractérisés en ce que :
- l'enchaînement A-D comporte un groupe Ze et un groupe Wiβ, tandis que l'enchaînement B-C comporte un groupe Ze et un groupe Wiα,
- ou l'enchaînement A-D comporte un groupe Ziβ et un groupe We, tandis que l'enchaînement B-C comporte un groupe Ze et un groupe Wiα,
- ou les enchaînements A-D et B-C comportent chacun un groupe Ziβ et un groupe We.

Des composés de formule (Ic) préférés sont ceux caractérisés par les formules suivantes :

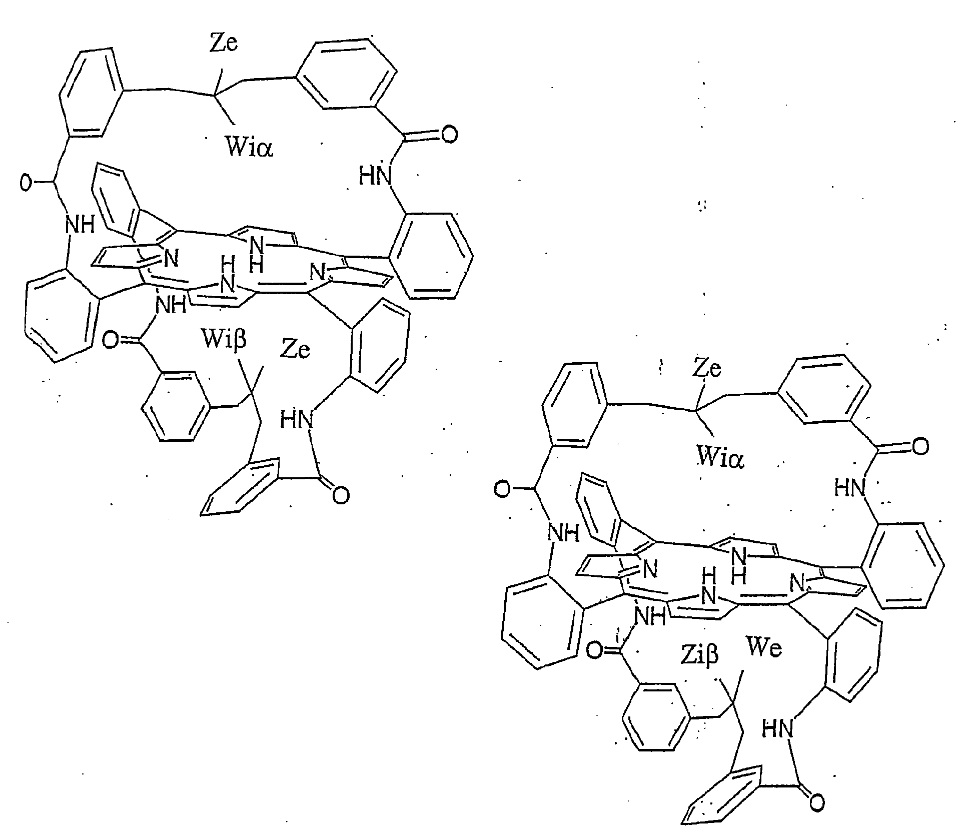

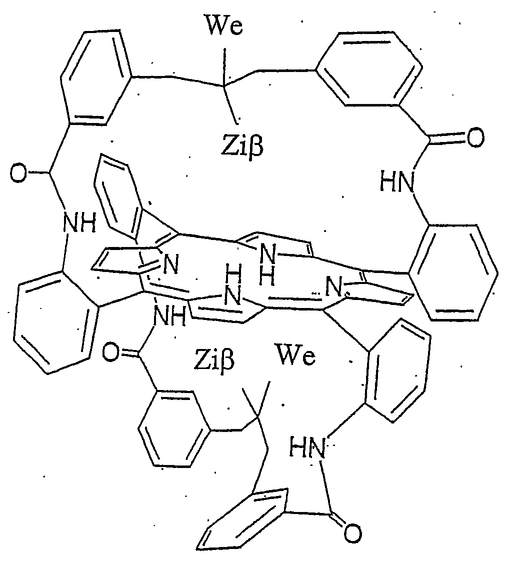

L'invention concerne également les composés de formule (I), et plus particulièrement ceux de formule (Ia), (Ib), et (Ic), tels que définis ci-dessus, dans lesquels Z représente un groupe CH₂NR₁R₂, dans lequel l'un au moins de R₁ et R₂ représentent un anticorps déterminé, le cas échéant lié à la partie CH₂N dudit groupe via un espaceur.

De tels anticorps peuvent être choisis parmi ceux cités dans le Symposium Roche, du jeudi 7 juin 2001, Paris, EUROCANCER 2001, notamment parmi les anticorps suivants :
- J591 : IgG2A murine, anti PSMA (Prostate Spécifique Membrane Antigen), exprimé sur les cellules carcinomateuses humaines de prostate,
- B4 : IgG1 murine, anti CD19, exprimé sur les cellules de lymphome Ramos et Daudi,
- HuM195 : IgG1 humanisée, anti CD33 exprimé sur des cellules humaines de leucémie HL60,
- 3F8: IgG3 murine, anti CD2 exprimé sur des cellules humaines de neuroblastome NMB7,
- Herceptin, trastuzumab : IgG1 humanisée, anti HER2 exprimé par les cellules humaines de carcinome su sein MCF7 et de l'ovaire SKOV3,
- 35A7 : dirigé contre l'antigène carcino-embryonaire (CEA),
- Basiliximab, Simulect : entièrement chimérique, anti-CD25, utilisé dans la prévention du rejet de la greffe du rein,
- Gentuzuma ozogomicin : entièrement humanisé, anti-CD33,
- Rituximab, Tositumomab : chimérique, anti-CD20, antigène exprimé dans plus de 95% des lymphocytes néoplasiques,
- BL22 : anti-CD22,
- SGN-10 : anti-Le Y, exprimé par différents types de carcinome, en particulier par les cellules épithéliales digestives et par les cellules acineuses pancréatiques.

A titre d'illustration, l'espaceur susmentionné est choisi parmi les groupes de formules suivantes : 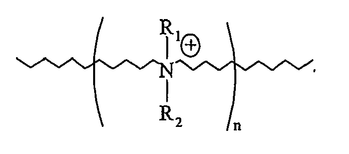 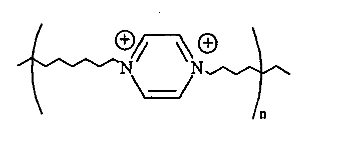 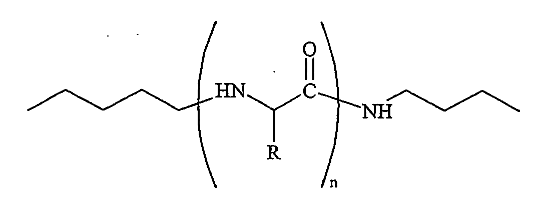 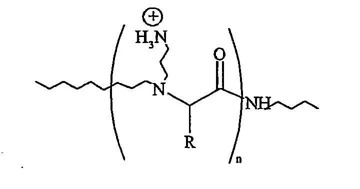 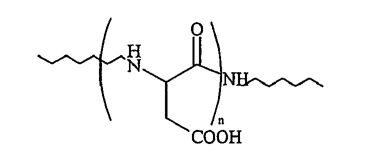 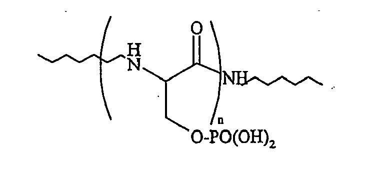 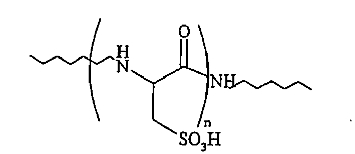 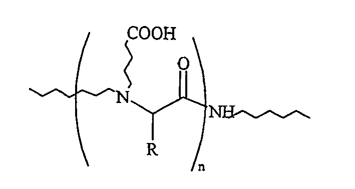 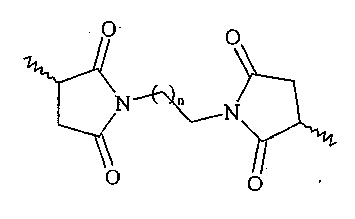 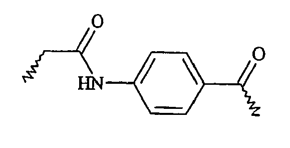 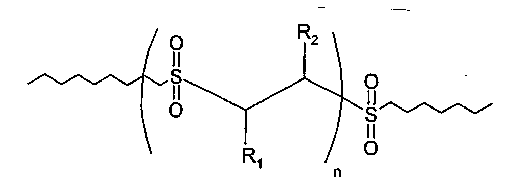 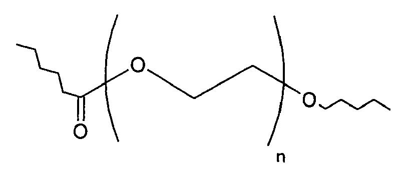 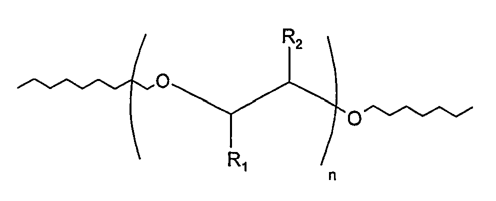 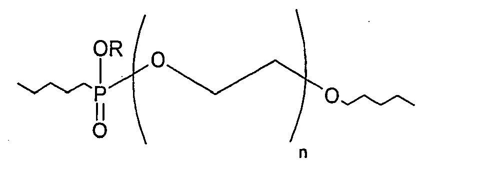 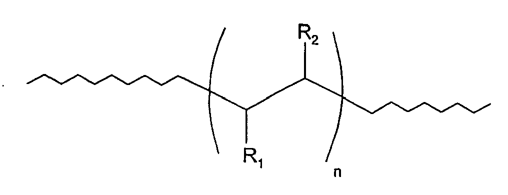 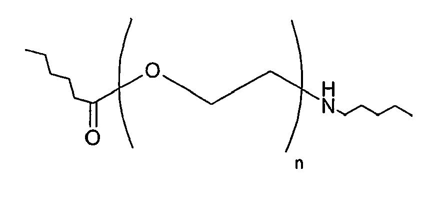 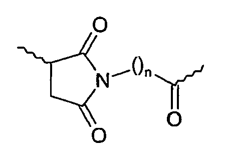 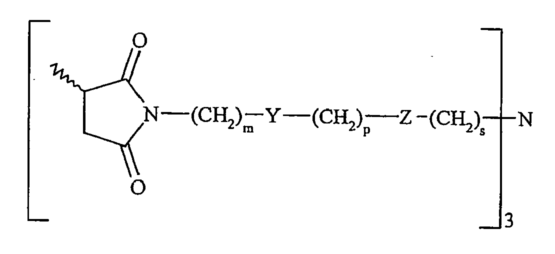 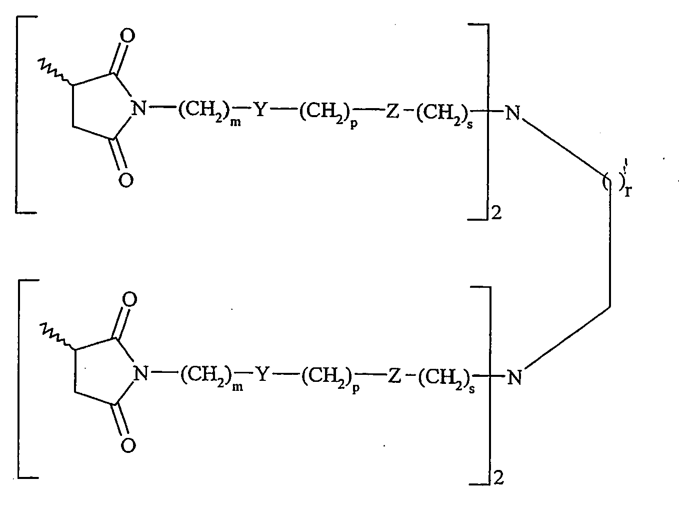 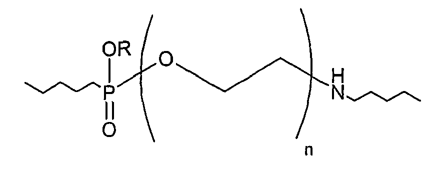 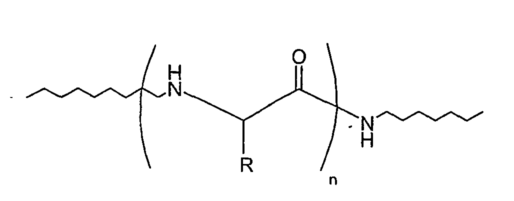 dans lesquelles n et r représentent un nombre entier variant de 1 à 10, R₁ et R₂ représentent, indépendamment l'un de l'autre, H, ou un groupe alkyle linéaire, ramifié, ou cyclique, de 1 à 8 atomes de carbone, ou un groupe aryle ou alkylaryle, R représente une des chaînes latérales des 20 acides aminés naturels, Y et Z représentent des hétéroatomes tels que O ou S, m, p et s, indépendamment les uns des autres, représentent 0 ou un nombre entier variant de 1 à 10.

L'invention concerne également les complexes entre un composé tel que défini ci-dessus, et un radioélément choisi parmi les émetteurs α, ou un élément métallique divalent ou trivalent.

L'invention a plus particulièrement pour objet les complexes susmentionnés entre un composé tel que défini ci-dessus, et un radioélément émetteur α choisi parmi le bismuth-212 ou -213, l'actinium-225, ou l'astate-211.

L'invention concerne plus particulièrement encore les complexes susmentionnés entre un composé tel que défini ci-dessus, et un élément métallique divalent ou trivalent choisi parmi Y(III), In(III), Cd(II), Mg(II), Mn(III), Fe(III), B(III) et les lanthanides.

Dans les complexes de l'invention, les métaux sont situés au centre du noyau porphyrinique des composés susmentionnées, mais pas obligatoirement dans le plan de la porphyrine, et sont associés aux atomes d'azote dudit noyau par des liaisons covalentes, dont deux sont de type datives.

L'invention a également pour objet toute composition pharmaceutique caractérisée en ce qu'elle comprend un complexe tel que défini ci-dessus, en association avec un véhicule pharmaceutiquement acceptable.

Avantageusement les compositions pharmaceutiques selon l'invention se présentent sous une forme administrable par voie intraveineuse.

De préférence, les compositions pharmaceutiques susmentionnées sont caractérisées en ce que la posologie est d'environ 15 à 50 mCi par patient répartis en 3 à 6 fractions sur 2 à 4 jours.

L'invention concerne également l'utilisation de complexes tels que définis ci-dessus pour la préparation d'un médicament destiné au traitement des cancers, ou pour la préparation de compositions destinées à l'imagerie médicale.

L'invention a plus particulièrement pour objet l'utilisation de complexes tels que définis ci-dessus, pour la préparation d'un médicament destiné au traitement des cancers à petites cellules tumorales, tels que la leucémie myéloïde aiguë, les lymphomes non hodgkidiens, les dysplasies bronchopulmonaires, les cancers du sein métastatiques, les cancers colorectaux, les lymphomes, et les pathologies dans lesquelles sont impliqués les motifs antigéniques suivants : CD52, CD22, CD20, HLA-DR, CD33, LE-Y, Ep-CAM, ACE, CAN, EGFR, KSA, VEGF, HER2, GD2, tenascin.

L'invention concerne également un procédé de préparation des composés de formule (I) susmentionnés, caractérisé en ce qu'il comprend les étapes suivantes :
- traitement du composé de formule (II) suivante 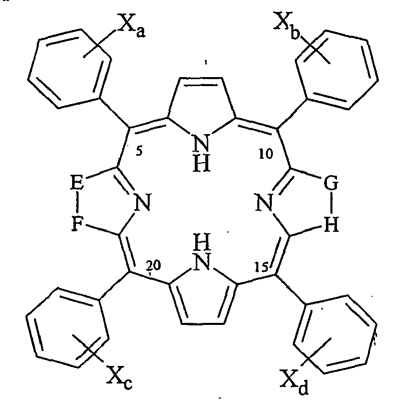 dans laquelle Xₐ, X_{b}, X_{c}, et X_{d}, représentent NH₂, OH, COOH ou CH₂Cl, et E, F, G, et H étant définis ci-dessus, ledit composé étant tel que :
   - Xₐ, X_{b}, X_{c}, et X_{d}, sont en position α dans le cas de la synthèse de composés de formule (Ia),
   - Xₐ, et X_{c}, sont en position α, et X_{b} et X_{d} sont en position β dans le cas de la synthèse de composés de formule (Ib),
   - Xa, et Xd, sont en position β, et X_{b} et X_{c} sont en position α dans le cas de la synthèse de composés de formule (Ic),
      avec un composé de formule Yₐ-C₆H₄-CH₂Cl dans laquelle Yₐ représente, COOH, CH₂Cl, NH₂, ou OH, respectivement,
- une étape de traitement du composé obtenu lors de l'étape précédente avec un composé de formule Z-CH₂-W dans laquelle Z et W sont tels que définis ci-dessus, ce qui conduit à l'obtention d'un composé de formule (I) dont les différentes variantes de formules Ia (Ib), et (Ic) sont séparées par purification, notamment par chromatographie basse pression sur gel de silice, ou HPLC préparative.

Les complexes susmentionnés sont obtenus par mise en présence des composés de formule (I) avec un radioélément tel que défini ci-dessus.

La description sera davantage illustrée à l'aide de la description détaillée qui suit de composés particuliers de l'invention, et de leur procédé d'obtention.

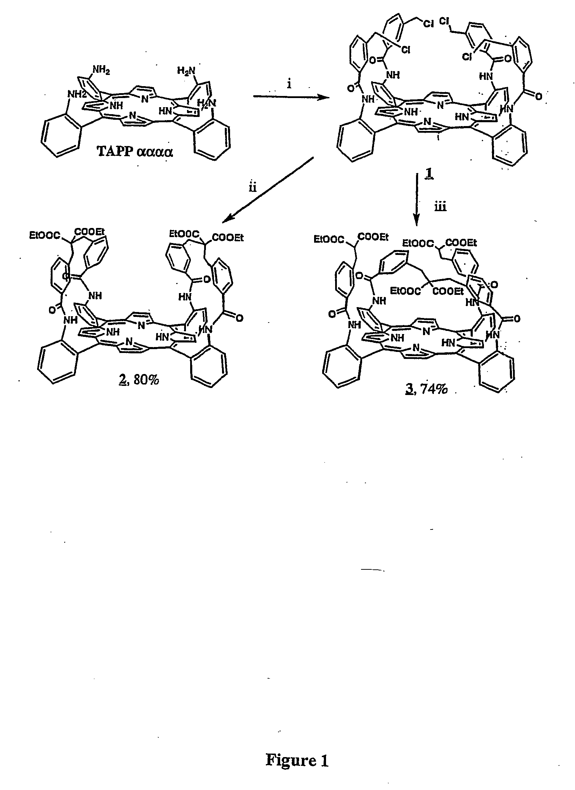

S'agissant du bismuth, compte tenu des propriétés de ce métal, à savoir son caractère azophile et oxophile et son nombre de coordination élevé (jusqu'à neuf atomes), les Inventeurs ont synthétisé des modèles correspondant le mieux possible aux exigences du métal. En effet, les atomes d'azote provenant du cycle porphyrinique interviennent dans la complexation du métal et les anses apportent au métal des groupes possédant des atomes d'oxygène. De plus, il faut noter que le ligand forme une cage capable d'accueillir et de stabiliser le métal. La figure 1 représente le squelette de base de ligands, ainsi que la méthode de synthèse de ces derniers.

L'apport de ce type de porphyrine par rapport aux porphyrines à piquets (Buckingham, D.; Clarck, C.; Webley, W.J. Chem. Soc. Chem. Com. 1981, 192*,* Michaudet, L.; Richard, P.; Boitrel, B. Chem. Commun. 2000, 1589-1590*, Michaudet, L. thèse de doctorat de l'Université de Bourgogne,* ***7*/*12*/*****2000**, Dijon*) réside dans la pré-organisation de l'anse (ou des anses). Le fait que les piquets ou les anses soient préorganisés permet de disposer d'un groupe de type carboxylique (acide, ou ester) juste au dessus du métal. De plus, grâce à la modularité de la synthèse, il est envisageable de faire varier le nombre de groupes coordonnants.

Ce schéma, bien que représentant la base de la présente invention, n'est pas applicable et a déjà été publié (Didier, A.; Michaudet, L.; Ricard, D.; Baveux Chambenoit, V.; Richard, P.; Boitrel, B. Eur.J. Org. Chem. 2001, 1917-1926), et ce pour deux raisons différentes. D'une part, après saponification des fonctions ester (et par conséquent décarboxylation), il n'y a aucun contrôle de la position de la fonction acide résiduelle. D'autre part, ces porphyrines ne possèdent pas de point de fonctionnalisation ultérieure, nécessaire pour le greffage sur un anticorps, ou pour rendre le composé hydrosoluble.

Par contre, ce type de ligand possède une structure très rigide, avec une géométrie prédéterminée, qui devrait accroître la stabilité du complexe forme. C'est en effet pour des raisons de stabilisation de l'élément métallique que la synthèse du cyclohexylbenzyl DTPA avait été mise au point, ce ligand présentant une nature plus rigide que le DTPA *(*Brechbiel, M.; W.; Gansow, O. A. J. Chem. Soc., Perkin Trans. I 1992, 1173*)*.

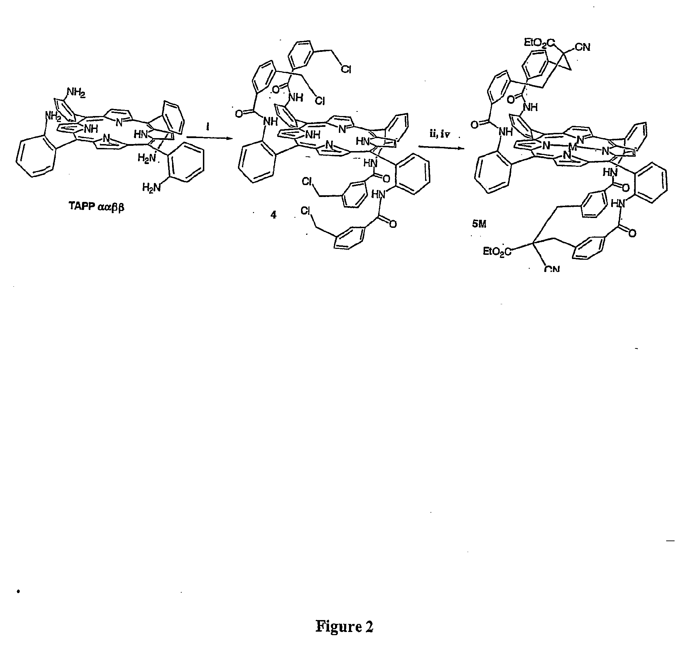

Afin d'éviter l'obtention de deux produits différents tels que représentés dans la figure 1, la même stratégie de synthèse a été appliquée à l'isomère ααββ (figure 2).

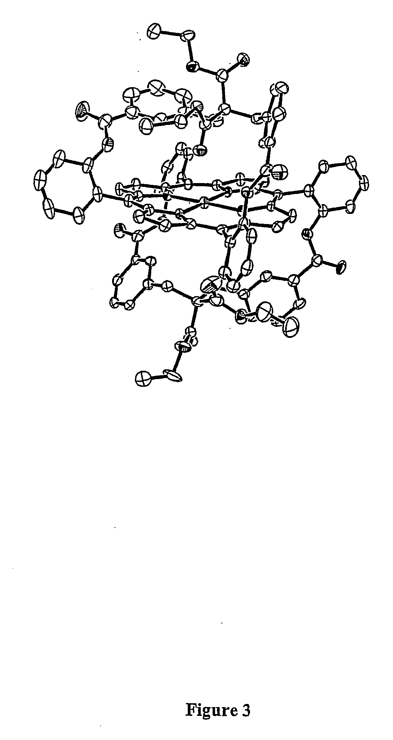

L'obtention d'une structure radio-cristallographique montre clairement que le groupement éthoxycarbonyle dirigé vers l'intérieur de la porphyrine est convenablement maintenu au-dessus du métal (figure 3).

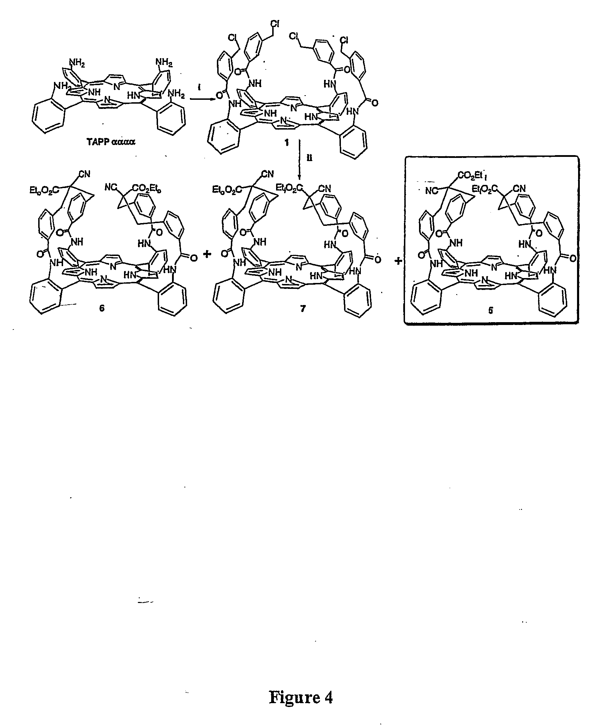

L'invention consiste à utiliser le cyano-acétate d'éthyle (NC-CH₂-CO₂Et) au lieu du malonate d'éthyle lors de la synthèse décrite dans la figure 1, et à l'appliquer, en plus de l'isomère αααα, aux isomères ααββ et αβαβ. Dans le cas de l'isomère αααα, trois porphyrines sont ainsi obtenues, parmi lesquelles on peut purifier la porphyrine **5** qui possède la fonction ester orientée vers l'intérieur (figure **4**).

Cette orientation de la fonction ester permet de rendre ces composés utilisables pour une coordination de métaux tels que le bismuth (III) ou les lanthanides. Par spectroscopie du proton, il est immédiat d'attribuer à une molécule la structure **5** du fait de sa symétrie et du blindage significatif subi par les groupes éthyles orientés vers l'intérieur de la cavité. En effet, par analogie avec le spectre de spectroscopie RMN du composé **2**, les composés **5** et **6** sont facilement discernables. De plus, le composé **7** représente un ligand de même conformation, mais avec un seul groupement éthoxycarbonyle pour coordonner le métal.

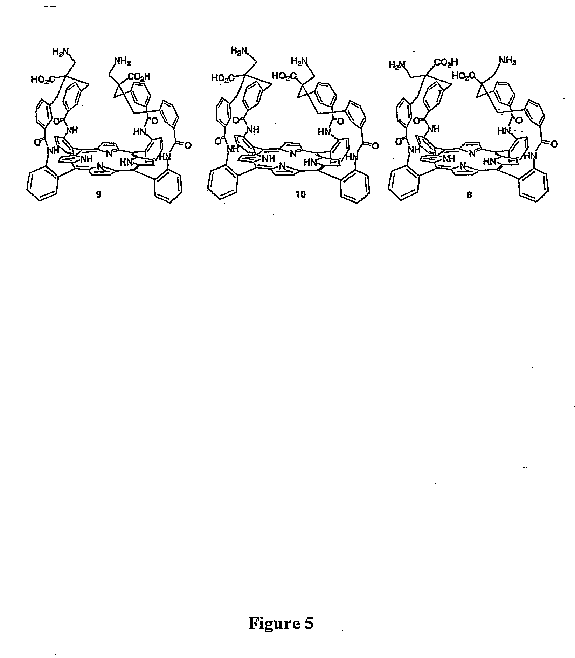

Donc, après saponification dans un premier temps, et réduction de la fonction CN en CH₂-NH₂ dans un deuxième temps, on obtient des produits dont la conformation est parfaitement connue et qui possèdent deux points de fonctionnalisation ultérieure (figure 5).

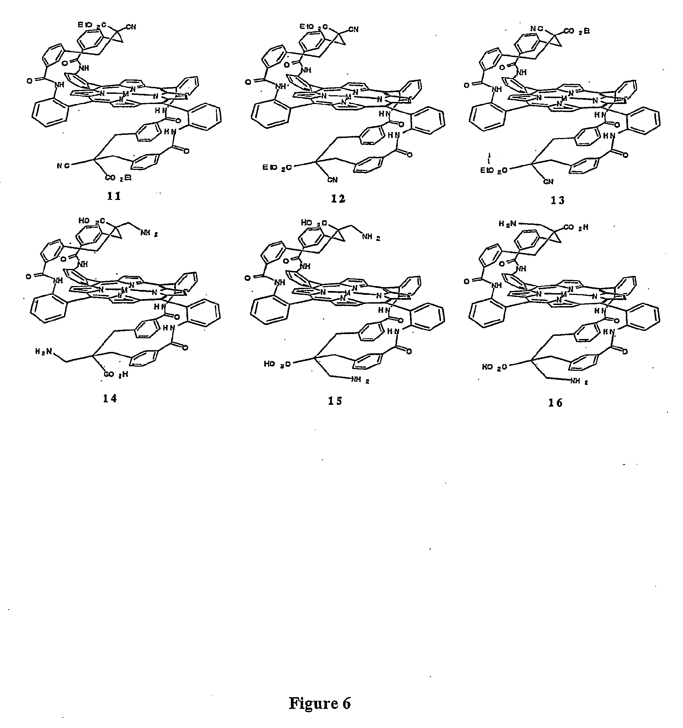

De même que décrit pour la figure 2, cette variante appliquée à l'atropisomère ααββ donnera lieu aux six ligands représentés sur la figure 6.

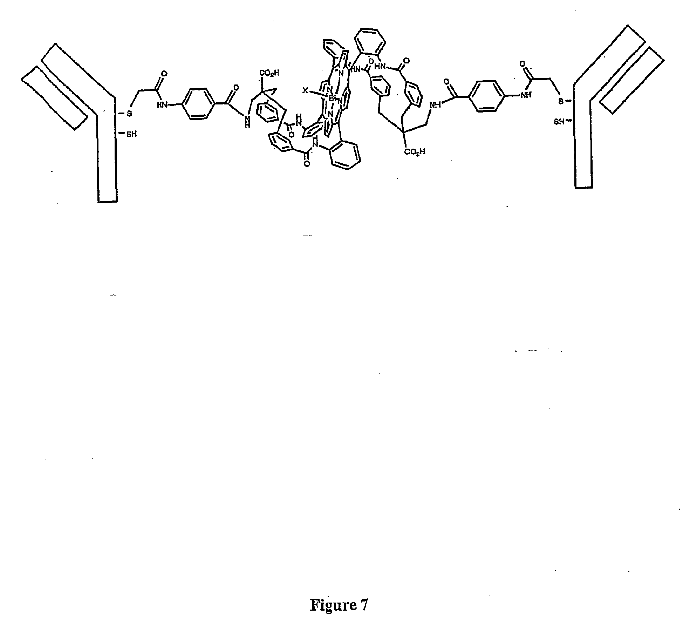

L'intérêt des porphyrines, représentées dans la figure 6, réside dans l'identité de leurs deux faces tant pour la coordination du métal que pour le greffage à un anticorps. Cette structure est utilisable pour la construction d'anticorps monoclonaux bispécifiques. Ces derniers résultent de l'assemblage de deux « demi-anticorps » sur un espaceur bifonctionnel (tel qu'un dérivé de type bis-maléimide). La porphyrine **16** peut être considérée dans ce cadre à la fois comme élément complexant et comme élément pontant comme représenté schématiquement sur la figure 7.

Notons que la porphyrine a été greffée sur deux fragments Fab' via un espaceur bifonctionnel appelé SIAB pour (N-succinimidyl(4-iodoacétyl)-aminobenzoate. Ces deux fragments peuvent être de spécificité différente afin d'améliorer la spécificité de reconnaissance.

Notons également que deux fonctions thiols sont présentées sur le fragment Fab', et que par conséquent différents schémas de connexion sont possibles entre la porphyrine et l'anticorps.

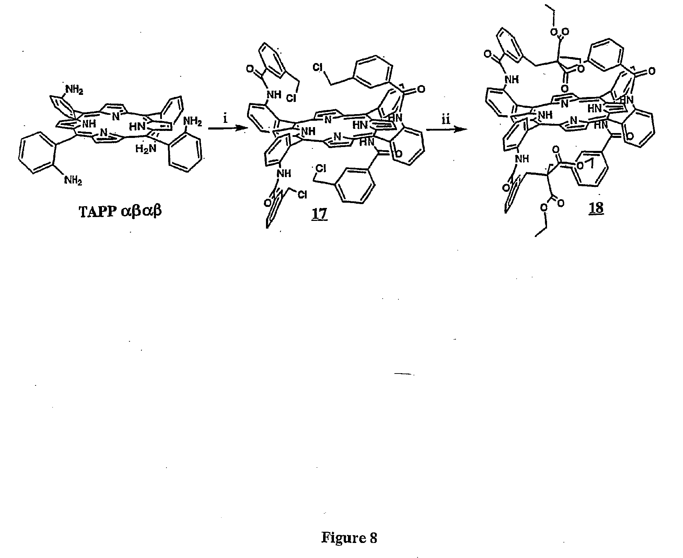

Enfin, le fait d'obtenir le composé **3** (figure 1) à partir de l'atropisomère αααα démontre que la même séquence réactionnelle appliquée à l'atropisomère αβαβ donne lieu au composé **18**, de type *bis-ansa* (figure 8). Ce type de composé est intéressant pour interdire toute interaction intermoléculaire comme la formation de dimères telle que décrite pour des porphyrines à piquets aliphatiques (Michaudet et al., 2000, susmentionné).

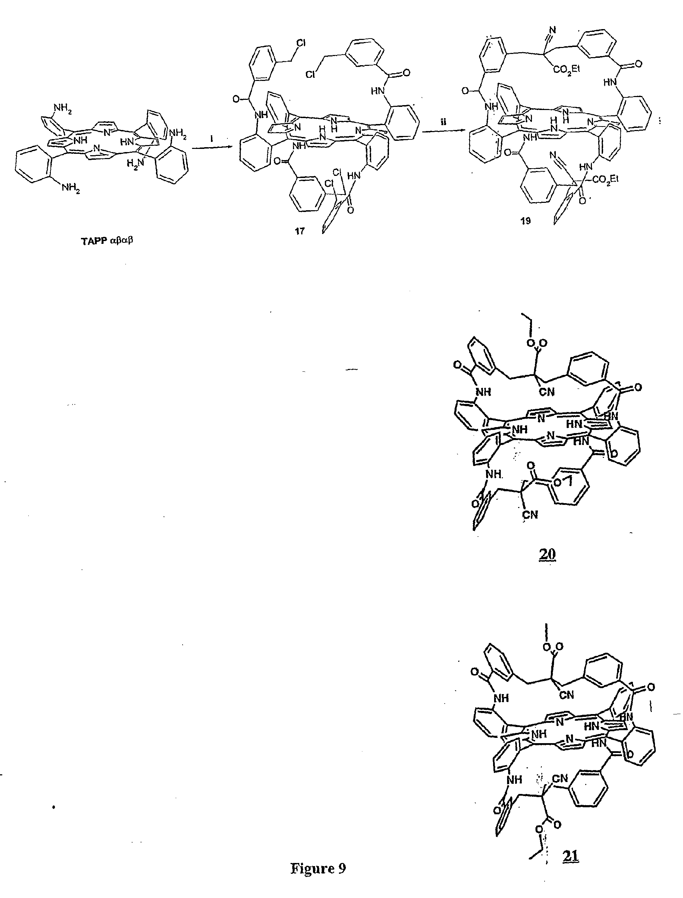

Une modélisation par dynamique moléculaire de la porphyrine **18** montre que la pré-organisation de cette superstructure dirige parfaitement une des deux fonctions carbonyle (appartenant à l'ester) vers le centre de coordination. Ce point signifie que l'on peut de nouveau différencier le groupement éthoxycarbonyle « intérieur » de celui « extérieur », et donc que si l'étape ii) est réalisée avec du cyanoacétate d'éthyle, on obtient de nouveau trois porphyrines **19, 20** et **21** (figure 9).

### PARTIE EXPERIMENTALE

### α-5,10,15,20-Tétrakis{2-[(3-chlorométhyl)benzoylamido]phényl}porphyrine : 1

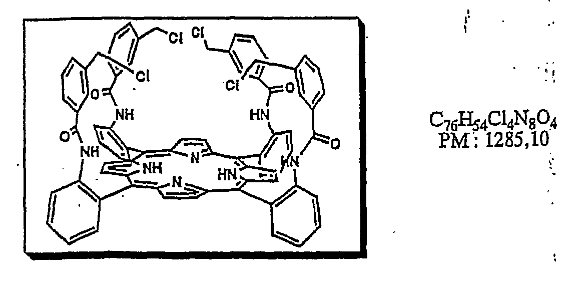

Dans un bicol de 100 mL, on introduit 0,2 g de TAPP 4.0 (0,29 mmol), 0,5 mL de triéthylamine et 20 mL de THF, sous argon. 0,34 mL (2,3 mmol) du chlorure de l'acide 3-(chlorométhyl) benzoïque sont ajoutés au moyen d'une seringue. La réaction est réalisée à température ambiante pendant 12 heures, puis le mélange réactionnel est évaporé. On purifie par chromatographie sur colonne de silice, le produit désiré est élué avec un mélange de méthanol dans du dichlorométhane (0,1%) puis isolé avec un rendement de 81 % (0,3 g, 0,23 mmol).
Analyse élémentaire : C₇₆H₅₄N₈Cl₄O₄, calculée (%) : C, 71,03 ; H, 4,24 ; N, 8,72 ; trouvée (%) : C, 70,89 ; H, 4,11 ; N, 8,83
Spectrométrie de masse (FAB) : m/z = 1284,9 [M]⁺.
Infra-rouge (KBr, cm⁻¹) : 1680 (C=O)_{amide'} 3415 (NH)
Spectrométrie de masse HRMS : m/z calculée = 1304,2920 pour C₅₆H₄₆N₈O8Na m/z mesurée =1304,2909
RMN ¹H (δ ppm, CDCI₃, 300K : 8,99 (s, 8H, β-pyr.); 8,89 (d, J = 8,3 Hz, 4H, aro.) ; 8,02(dd, J = 1,3 Hz, J =7,5 Hz, 4H, aro.) ; 7,92 (td, J = 1,3 Hz, J = 8,3 Hz, 4H, aro.) ; 7,81 (s, 4H, -NHCO) ; 7,59 (td, J = 0,9 Hz, J = 7,6 Hz, 4H, aro.) ; 6,52 (large s, 4H, aro _{piq.}) ; 6,51 (d, J = 8,1 Hz, 4H, aro_{piq}) ; 6,40 (d, J = 7,7 Hz, 4H, aro_{piq}); 6,40 (d, J = 7,7 Hz, 4H, aro_{piq}); 6,00 (t, J = 7,7 Hz, 4H, aro_{piq}); 3,23 (s, 8H, -CH₂-) ; -2,47 (s, 2H).
RMN ¹³C (δ ppm, CDCI₃, 300K) : 165,2 ; 138,8 ; 137,5 ; 135,6 ; 135,2 ; 132,4 ; 131,3 ; 131,0 ; 130,6 ; 128,4 ; 126,6 ; 126,0 ; 123,9 ; 121,3 ; 115,5 , 44,6.

### α-5,10 : α-15,20- Bis-{2,2' -[3,3'-(2,2-(diéthoxycarbonyl)propane-1,3-diyl) dibenzoylamido]diphényl}porphyrine : 2

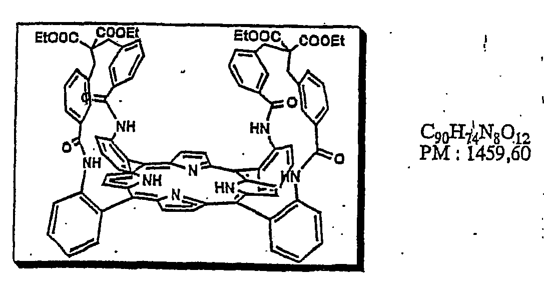

Dans un tricol de 50 mL, sous argon, on dissout 18 mg (0,8 mmol) de sodium dans 5 mL d'éthanol absolu. Après ½ heure, on ajoute, à l'aide d'une seringue, le malonate d'éthyle (118µl, 0,8 mmol). 50 mg de **1** (0,04 mml), sont préalablement dissous dans 10 mL de THF, puis ajoutés goutte à goutte dans le mélange réactionnel. Le brut est évaporé au bout de 24 heures, puis déposé sur colonne de silice. Le produit est élué avec un mélange de MeOH/CH₂Cl₂ et obtenu avec un rendement de 80% (46 mg, 0,03 mmol).
Analyse élémentaire : C₉₀H₇₄N₈O₁₂•CH₂Cl₂, calculée (%) : C, 70, 76 ; H, 4,96 ; N, 7,25 trouvée (%) : C, 70,68 ; H, 4,06 ; N, 6,93
Infra-rouge (KBr, cm⁻¹) : 1726 (C=O) ₑₛₜₑᵣ, 1683 (C=O) _{amide} 3417 (NH)
Spectrométrie de masse (FAB) : m/z = 1459,1 [M]⁺
Spectrométrie de masse HRMS : m/z calculée = 1481,5335 pour C₉₀H₇₄N₈NaO₁₂ ; m/z mesurée = 1481,5324
RMN ¹H (δ ppm, CDCl₃, 300K) : 8,92 (s, 4H, β-pyr) ; 8,87 (s, 4H, β-pyr.) ; 8,69 (d,J = 8,4 Hz, 4H, aro.) ; 7,91 (td, J = 7,8 Hz, J = 1,3 Hz, 4H, aro.) ; 7,79 (dd, J = 7,5 Hz, J =1,2 Hz, 4H, aro.) ; 7,49 (td, J = 7,5, 4H, aro.) ; 7,41 (s,4H, -NHCO) ; 7,07 (d, J = 7,9 Hz 4H, aro _{piq}) ; 6,65 (d, J = 7,5 Hz, 4H, aro _{piq.}) ; 6,59 (s, 4H, aro _{piq.}); 6,39 (t, J = 7,7 Hz, 4H, aro_{piq.}) ; 3,98 (q, J = 6,9 Hz, 4H, -**CH**₂CH₃) ; 3,53 (q, J = 7,1 Hz, 4H, -**CH**₂CH₃) ; 2,38 (d, J = 13,8,4H, -CH₂-) ; 2,19 (d, J = 13,8 Hz, 4H, -CH₂-) ; 1,09 (t, J = 6,9Hz, 6H, -CH₂**CH**₃) ; 0,64 (t, J = 7,1 Hz, 6H, -CH₂**CH**₃) ; -2,95 (s, 2H).
RMN ¹³C (δ ppm, CDCl₃, 300K) : 170,9 ; 170,5 ; 166,7 ; 138,3 ; 136,5 ; 135,9 ; 134,7 ; 133,2 ; 132,1 ; 130,3 ; 128,6 ; 127,8 ; 126,3 ; 123,7 ; 122,7; 115,4 ; 31,7; 61,7 ; 61,4 ; 40,7 ; 14,4 ; 13,8.

### α-5,15-{2,2'-[3,3'-(2,2-(diéthoxycarbonyl)propane-1,3-diyl)dibenzoylamido] diphényl}: α-10,20-Bis-{2,2'-[3,3'-(1,1-(diéthoaycarbonyl)ethane-2yl) benzoylamido]phényl} porphyrine : 3

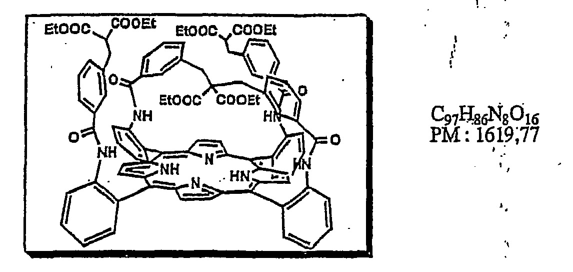

Le même mode opératoire que celui adopté pour synthétiser la molécule précédente est mis en oeuvre. Au départ de 0,89 de sodium (40 mmol) et 5,9 mL de malonate d'éthyle (40 mmol) dans 35 mL d'éthanol absolu, on ajoute 50 mg (0,04 mmol) de porphyrine **1** dissoute dans 10 mL de THF. Le brut est chromatographié sur une colonne de silice et le produit désiré est élué avec un mélange de pentane/chloroforme (5/100) avec un rendement de 74% (47 mg, 0,03 mmol).
Analyse élémentaire : C₉₇H₈₆N₈O₁₆•H₂O, calculée (%): C,71,14 ; H,4,42; N, 6,84 ; trouvée (%) : C, 69,92 ; H, 4,25 ; N, 6,63
Spectrométrie de masse (MALDITOF) : m/z = 1619,3 [M]^{+.}
Infra-rouge (KBr, cm⁻¹) : 1732 (C=O) _{ester'} 1682 (C=O) _{amide'} 3416 (NH)
RMN¹H (δ ppm, CDCl₃, 320K) : 9,08 (d, J = 8,44 Hz, 2H, aro) ; 9,06 (d, J = 4,7 Hz, 4H, β-pyr.) ; 8,95 (d, J=4,7 Hz, 4H, β-pyr) ; 8,71 (d, J = 8,4 Hz, aro.) ; 8,56 (large s, 2H, -NHCO) ; 8,04 (dd, J =1,1 Hz, J = 6,9 Hz, 2H, aro.) ; 7,94 (large t, J = 6,6 Hz, 4 H, aro., - NHCO) ; 7,85 (td, J = 1,3 Hz, J = 8,2 Hz, 2H, aro.) ; 7,69 (d, J = 7,7 Hz, 2H, aro_{piq}.) ; 7,65 (dd, J = 1,3 Hz, J = 7,7 Hz, 2H, aro.) ; 7,59 (t, J = 7,2 Hz, 2H, aro.) ; 7,55 (s, 2H, aro_{piq}.) ; 7,47 (t, J = 7,5 Hz, 2H, aro.) ; 6,97 (t, J = 7,7 Hz, 2H, aro_{piq}.) ; 6,50 (d, J = 7,8 Hz, 4H, aro_{piq}.) ; 6,44 (d, J = 5,7 Hz, 2H, aro_{piq}.) ; 5,88 (t, J = 7,4 Hz, 2H, aro_{piq}.), 4,84 (s, 2H, aro_{piq}.) ; 4,09 (m, 8H, -CH₂-CH₃) ; 3,38 (t, J = 7,6 Hz, 2H, -CH₂CH-) ; 2,81 (d, J = 7,6 HZ, 4H, -CH₂CH-) ; 1,63 (s, 4H, -CH₂-) ; 1,17 (t, J = 7,2 Hz, 12H, - CH₂CH₃) ; 0,95 (large s, 4H, -CH₂, CH₃) ; -0,6 (large s, 6H, -CH₂CH₃) ; -2,25 (s, 2H).
RMN ¹³C (δ ppm, CDCl₃, 300K) : 168,8 ; 168,1 ; 166,6 ; 164,6 ; 139,2 ; 138,9 ; 138,0 ; 137,7 ; 136,1 ; 135,4 ; 135,2 ; 133,7 ; 132,9 ; 132,5 ; 131,9 ; 131,4 ; 130,6 ; 130,3 ; 128,8 ; 128,7 ; 128,5 ; 127,7 ; 125,8 ; 123,9 ; 123,5 ; 122,6 ; 120,9 ; 116,5 ; 115,1 ; 62,0 ; 53,6 ; 42,0 ; 34,1 ; 14,2 ; 12,0.

### α-5,10 : β-15,20-Tétrakis{2-[(3-chlorométhyl)benzoylamido]phényl} porphyrine : 4

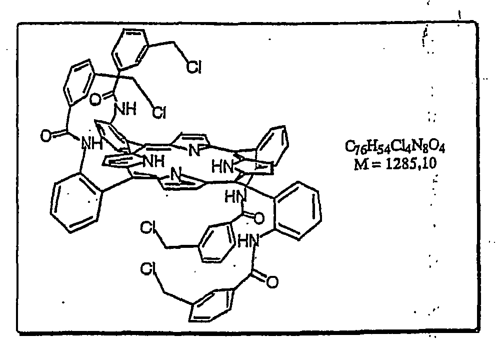

Dans un bicol de 250 mL, on introduit 0,674 g (1 mmol) de TAPP ααββ, 2,22 mL (16 mmol) de triéthylamine et 100 mL de THF, sous argon. 0,71 mL (5 mmol) du chlorure de l'acide 3-(chlorométhyl)benzoïque dissous dans 10 mL de THF sont ajoutés goutte-à-goutte. La réaction est réalisée à 0°C pendant 3 heures, puis le mélange réactionnel est évaporé. On purifie le résidu par chromatographie sur colonne de silice, le produit est élué par du dichlorométhane pur, puis isolé avec un rendement de 86% (1,10g).
*RMN¹H 500 MHz (δppm, CDCl₃, 300K)* : -2,52 (s, 2H, NH_{pyr}) ; 3,52 (d, 4H, *Jo* = 12,1 Hz, (CH₂)_{benz}) ; 3,55 (d, 4H, *Jo* = 12,1 Hz (CH₂)_{benz}) ; 6,39 (t, 4H, *Jo* = 7,7 Hz, aro_{piq}) ; 6,52 (d, 4H, *Jo* = 8,3 Hz, aro_{piq}) ; 6,55 (s, 4H, aro_{piq}) ; 6,74 (d, *4*H, *J₀* = 7,5 Hz, aro_{piq}) ; 7,61 (t, 4H, *Jo* = 7,5 Hz, aro) ; 7,66 (s,4H, NHCO) ; 7,93 (t,4H, *Jo* = 8,3 Hz, aro) ; 8,07 (d, 4H, *Jo* = 7,3 Hz, aro) ; 8,90 (d, 4H, *Jo* = 8,3 Hz, aro) ; 8,99 (s, 4H, β-pyr) ; 9,00 (s, 4H, β-pyr).
RMN ¹³C 125 MHz (δ ppm, CDCI₃, 300K) : 44,8 ; 115,4 ; 121,5 ; 123,9 ; 126,3 ; 126,6 ; 128,7 ; 130,7 ; 131,3 ; 135,2 ; 135,5 ; 137,6 ; 138,8 ; 165,2.
UV-vis (CH₂Cl₂, λ/nm (10⁻³.ε, M⁻¹.cm⁻¹)) : 422 (363,8) ; 515 (20,6) ; 549 (5,1) ; 589 (6,2) ; 646 (2,6).
*Spectrométrie de masse* (SMHR, LSIMS) calculée m/z = 1305,2920 [M+Na]⁺ pour C₇₆H₅₄Cl₄N₈NaO₄, trouvée 1305,2899.
*Analyse élémentaire* : pour C₇₆H₅₄Cl₄N8O₄, calculée (%) : C, 71,03 ; H, 4,24 ; N, 8,72 ; trouvée (%) : C, 70,62 ; H, 4,19 ; N, 8,94.
*Infrarouge* (KBr, υ cm⁻¹) : 3420 5NH) ; 1684 (CO).

### α-5,10:β-15,20-Bis{2,2'-[3,3'-(2,2'-(diéthoxycarbonyl)propane-1,3-diyl) dibenzoylamido]diphényl}porphyrine : 5

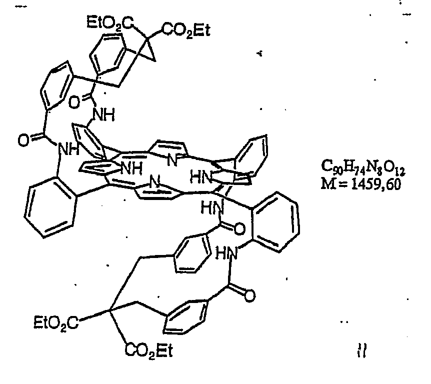

Dans un tricol de 50 mL, sous argon, on dissout 0,19 mg (8,2 mmol) de sodium dans 30 mL d'éthanol absolu. Après 30 minutes, on ajoute à l'aide d'une seringue, le malonate d'éthyle (1,24 mL, 8,2 mmol). 0,35 g (0,27 mmol) de **4** sont préalablement dissous dans 20 mL de THF, puis ajoutés goutte-à-goutte dans le mélange réactionnel. Le brut est évaporé au bout de 2 heures, puis le résidu est déposé sur colonne de silice. Le produit est élué avec du dichlorométhane et obtenu avec un rendement de 75% (0,30g).
RMN¹H 500 MHz (δ ppm, CDCI₃, 323K) : -2,16 (s, 2H, NH_{pyr}),-0,03 (t, 6H, *J*= 7,0 Hz, CH₂(CH₃)ᵢ) ; 0,57 (d,4H, *Jo* = 13,7 Hz, (CH₂)_{benz}), 0,63 (t, 6H, *Jo* = 7,0 Hz, CH₂(CH₃)₀) ; 1,44 (d, 4H, *Jo* = 13,5 Hz, (CH₂)_{benz}) ; 2,46 (q, 4H, *Jo* = 7,0 Hz, (CH₂)ᵢCH₃) ; 3,29 (q, 4H, *Jo* = 7,0 Hz, (CH₂)ₒCH₃) ; 4,84 (s, 4H, aro_{piq}) ; 6,61 (d, 4H, *Jo* = 7,6 Hz, aro_{piq}) ; 6,93 (t, 4H, *Jo* = 7,6 Hz, aro_{piq}) ; 7,42 (s, 4H, NHCO) ; 7,51 (td, 4H, *Jo* = 7,6 Hz, *Jm* = 1,2 Hz, aro_{piq}) ; 7,55 (td, 4H, *Jo* = 7,3 Hz, *Jm* = 1,6 Hz, aro) ; 7,87 (td, 4H, *Jo =* 7,3 Hz, *Jm* = 1,0 Hz, aro) ; 7,89 (dd, 4H, *Jo* = 8,4 Hz, aro); 8,69 (s, 4H, β-pyr); 8,70 (dd, 4H, *Jo =* 8,4 Hz, *Jm* = 1,0 Hz, aro) ; 8,98 (s, 4H, β-pyr).
RMN¹³C125MHz (δ ppm, CDCl₃, 323K) : 12,9 ; 13,7 ; 40,5 ; 59,5 ; 60,3 ; 60,9 ; 115,2 ; 122,8 ; 123,9 ; 126,1 ; 127,2 ; 128,6 ; 130,3 ; 130,6 ; 132,4 ; 133,0 ; 133,4 ; 134,0 ; 134,9 ; 135,9 ; 139,0 ; 165,1 ; 168,9 ; 169,7.
UV-*vis* (CH₂Cl₂*,* λ/nm (*10⁻³*.ε *M¹.cm⁻¹*)) : 422 (433,5) ; 516 (17,4) ; 550 (4,8) ; 590 (5,6) ; 647 (1,4).
*Spectrométrie de masse* (FAB) : m/z = 1458,6 [M]⁺.
*Analyse élémentaire :* pour C₉₀H₇₄N₈O₁₂, calculée (%) : C, 74,06 ; H, 5,11 ; N, 7,68 ; trouvée (%) : C, 74,25 ; H, 5,35 ; N, 7,30.
*Infrarouge : (KBr, νcm⁻¹) :* 3426 (NH) ; 1728 (CO) ; 1686 (CO).

### 5Ni (structure radiocristallographique de la figure 3)

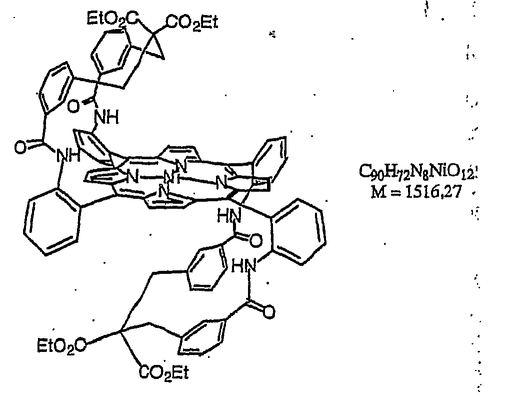

45 mg de **5** sont dissous dans 1,5 mL de pyridine. Un excès d'acétate de nickel est ajouté. La solution est portée à reflux pendant 1 heure, puis les solvants sont évaporés. Le résidu est dissout dans CH₂Cl₂, filtré et séché à nouveau. Après chromatographie sur colonne de silice (éluant : CH₂Cl₂/MeOH (98/2), le produit désiré est obtenu avec un rendement de 98%).
*RMN¹H MHz (δ ppm, CDCI3, 323K)* : 0,16 (t, 6H, *Jo* = 7,1 Hz, CH₂(CH₃)ᵢ) ; 0,70 (t, 6H, *Jo* = 7, Hz, CH₂(CH₃)ₒ) ; 1,01 (d, 4H, Jo = 13,7 Hz, (CH₂)_{benz}) ; 1,66 (d, 4H, *Jo* = 13,7 Hz, (CH₂)_{benz}) ; 2,81 (q, 4H, *J* = 7,6 Hzaro_{piq}) ; 3,41 (q, 4H, *J* = 7,1 Hz, (CH₂)oCH₃) ; 4,85 (s, 4H, aro_{piq}); 6,67 (d,4H, *J*= 7,6 Hz, aro_{piq}) ;6,96 (t, 4H, *J*= 7,6 Hz, aro_{piq}) ; 7,34 (s, 4H, NHCO) ; 7,47 (td, 4H, *Jo* = 7,6 Hz, *Jm* = 0,9 Hz, aro_{piq}) ; 7,59 (td, 4H, *Jo* = 7,7 Hz, *Jm* = 1,4 Hz, aro) ; 7,77 (dd, 4H, *Jo* = 7,6 Hz, *Jm* = 1,2 Hz, aro) ; 7,82 (td, 4H, *Jo* = 8,1 Hz, *Jm* = 1,2 Hz, aro) ; 8,65 (s, 4H, β-pyr) ; 8,70 (dd, 4H *Jo* = 8,1 Hz, *Jm* = 0,9 Hz, aro) ; 8,85 (s, 4H, β-pyr).
*RMN 13C 125 MHz (δppm, CDCI₃, 323K) :* 13,1 ; 13,8 ; 40,6; 60,6 ; 61,0; ;114,5 122,4 ; 123,8 ; 126,2 ; 127,3 ; 128,7 ; 130,3 ; 132,7 ; 133,0 ; 133,4 ; 133,9 ; 134,1 ; 136,0 ; 138,7 ; 164,7 ; 170,0.
*Spectrométrie de masse* (MALDI/TOF) m/z =1514,71 [M]⁺.
*Analyse élémentaire* : pour C₉₀H₇₂N₈NiO₁₂•H₂O, calculée (%) : C, 70,45 ; H, 4,86; N, 7,30 ; trouvée (%) : C, 70,54 ; H, 5,21 ; N, 7,06.
*Infrarouge (KBr, ν cm⁻¹) :* 3419 (NH) ; 1687 (CO).

### 5Zn

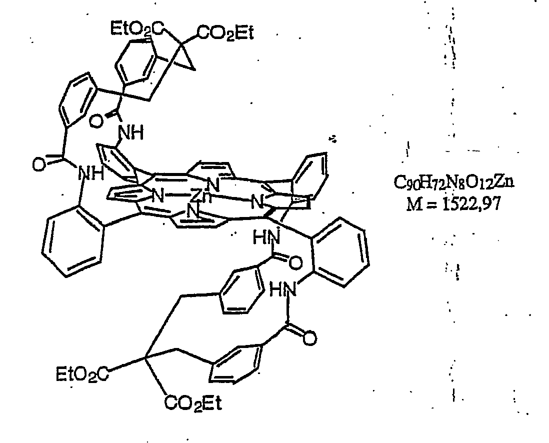

Ce complexe a été préparé à partir de **5**, selon la méthode suivante. 50 mg de porphyrine base sont dissous dans 10 mL d'un mélange CHCl₃/MeOH (2%). Un excès d'acétate de zind dihydraté et d'acétate de sodium sont ajoutés. La solution est portée à reflux pendant 1 heure, puis les solvants sont évaporés. Le résidu est dissous dans CH₂Cl₂, filtré et séché à nouveau. Après chromatographie sur colonne de silice (éluant : CH₂Cl₂/MeOH (97/3)), un produit rose-violet est isolé avec un rendement quantitatif (98%).
*RMN¹H 500 MHz (δppm, CDCI₃, 323K) :* -0,40 (d, 4H, *Jo* =13,0 Hz, (CH₂)_{benz}) ; 0,11 (t, 6H *Jo =* 7,1 Hz; CH₂(CH₃)i) ; 0,21 (t, 6H, *Jo* = 7,1 Hz, CH₂(CH₃)ₒ) ; 1,31 (d, 4H, *Jo* = 13,0 Hz, (CH₂)_{benz}-) ; 2,27 (q, 4H, *Jo* = 7,2 Hz, (CH₂)ᵢCH₃) ; 2,46 (q, 4H, *Jo* = 7,2 Hz, (CH₂) ₒCH₃) ; 3,82 (s, 4H, aro_{piq}) ; 6,54 (td, 4H, *Jo* = 7,7 Hz, *Jo* = 1,3 Hz aro_{piq}); 6,94(t, 4H, *Jo* = 7,7 Hz, aro_{piq}) ; 7,26 (s, 4H, NHCO) ; 7,55 (td, 4H, *Jo* = 7,6 Hz, *Jm* = 1,2 Hz, aro_{piq}) ; 7,62 (td, 4H, *Jo* = 8,1 Hz, *Jm* = 1,6 Hz, aro) ; 7,86 (td, 4H, *Jo* = 8,1 Hz, *Jm* = 1,5 Hz, aro) ; 7,99 (dd, 4H, *Jo* = 7,5 Hz, *Jm* = 1,2 Hz, aro) ; 8,69 (dd, 4H, *Jo* = 8,3 Hz, *Jm* = 0,9 Hz, aro) ; 8,79 (s, 4H, β-pyr) ; 9,03 (s, 4H, β-pyr).
*RMN¹³C 125 MHz (δppm, CDCI₃, 300K)* :13,1 ; 13,3 ; 40,5 ; 60,4 ; 115,8 ; 122,4 123,9 ; 124,7 ; 127,6 ; 128,8 ; 130,1 ; 132,7 ; 132,9 ; 133,0 ; 133,7 ; 133,9 ; 134,5 ; 139,2; 151,1 ; 151,9; 164,3 ; 168,2; 170,1.
*Spectrométrie de masse* (MALDI/TOF) : m/z =1522,01 [M+H]⁺.
*Analyse élémentaire:* pour C₉₀H₇₂N₈O₁₂Zn•2H₂O, calculée (%) : C, 69,34 ; H, 4,91 ; N, 7,19 ; trouvée (%) : C,69,09 ; H, 4,90 ; N, 7,43.
*Infrarouge (KBr, νcm⁻¹) :* 3420 (NH) ; 1728 (CO) ; 1683 (CO)

### Préparation des composés 11, 12 et 13 (voir Figure 6)

### Conditions expérimentales :

Dans un ballon de 250 mL, sous argon, on dissout 715 mg (31,1 mmol) de sodium dans 45 mL d'éthanol absolu. Après 1 heure, on ajoute à l'aide d'une seringue, le cyanoacétate d'éthyle (3,32 mL ; 31,1 mmol). La solution blanchit après quelques minutes. Le mélange est maintenu sous agitation pendant 1 heure. 400 mg (0,311 mmol) de 4 sont préalablement dissous dans 70 mL de THF, puis ajoutés'goutte à goutte dans le mélange réactionnel. Le brut est évaporé après 12 heures, puis le résidu est précipité par un mélange de dichlorométhane et de pentane. Le précipité est dissout dans un minimum de dichlorométhane afin d'être déposé sur colonne de silice. Une montée progressive à 0,2 % en méthanol permet d'obtenir les 3 produits attendus selon la position relative des groupes CN et CO₂Et. Plusieurs colonnes de chromatographie sont nécessaires afin d'obtenir ces 3 produits en pureté satisfaisante avec les rendements suivants : (8 mg, 2%), (70 mg, 17%) (150 mg, 36%).

Le rendement global de la réaction est évalué à 72 %.

### Caractérisation :

Produit obtenu à 2% :
**RMN ¹H (500 MHz, CDCl₃, 300 K) :** δ = 8,96 (s, 4H, β-pyr) ; 8,75 (d, *J* = 8,2 Hz, 4H) ; 8,62 (s, 4H, β-pyr) ; 7,87 (m, 8H) ; 7,71 (d, *J* = 7,9 Hz, 4H) ; 7,54 (t, *J* = 7,7Hz, 4H) ; 7,31 (s, 4H, NH) ; 7,08 (t, *J* = 7,7 Hz, 4H) ; 6,77 (d, *J*= 7,1 Hz, 4H) ; 4,42 (s, 4H, H_{2'}) ; 1,90 (q, *J*= 7,1 Hz, 4H, CH₂CH₃) ; 1,53 [d, *J* = 12,9 Hz, 4H, CH₂] ; -0,20 [br, 4H, CH₂] ; -0,56 (t, *J*= 7,1 Hz, 6H, CH₂CH₃) ; -1,96 (s, 2H). "
**ESI-HRMS :** calculé m/z = 1387,48063 [M+Na]⁺; trouvé 1387,4809.
UV-VIS (CH₂Cl₂, λ nm, 10⁻³ ε, M⁻¹.cm⁻¹) : 424 (221,0) ; 518 (14,2) ; 552 (3,5) ; 593 (3,8) ; 651 (0,9).

Produit obtenu à 17% :
**RMN ¹H (500 MHz, CDCl₃, 300 K) :** δ = 8,98 (s, 2H, β-pyr) ; 8,96 (s, 2H, β-pyr) ; 8,84 (d, *J* = 8,2 Hz, 2H) ; 8,70 (d, *J* = 4,7 Hz, 2H, β-pyr) ; 8,62 (d, *J* = 4,7 Hz, 2H, β-pyr) ; 8,52 (d, *J* = 8,2 Hz, 2H) ; 8,34 (d, *J* = 7,4 Hz, 2H) ; 7,92 (t, *J* = 7,9 Hz, 2H) ; 7,83 (t, *J* = 7,9 Hz, 2H) ; 7,75 (d, *J*= 7,9 Hz, 2H) ; 7,71 (t, *J*= 7,9 Hz, 2H) ; 7,61 (d, *J* = 7,7 Hz, 2H) ; 7,57 (s, 2H, NH) ; 7,54 (d, *J* = 7,4 Hz, 2H) ; 7,40 (t, *J* = 7,4 Hz, 2H) ; 7,08 (t, *J*= 7,7 Hz, 2H) ; 6,94 (t, *J*= 7,7 Hz, 2H) ; 6,89 (s, 2H, NH) ; 6,74 (d, *J*= 7,7 Hz, 2H) ; 6,38 (d, *J* = 7,7 Hz, 2H) ; 5,18 (s, 2H, H_{2'}) ; 3,58 (s, 2H, H_{2'}) ; 2,97 [q, *J*= 7,1 Hz, 2H, (C*H*₂)ₑCH₃] ; 2,10 [q, *J* = 7,1 Hz, 2H, (C*H*₂)ᵢCH₃] ; 1,77 [d, *J* = 12,9 Hz, 2H, CH₂] ; 1,39 [d, *J* = 12,9 Hz, 2H, CH₂] ; 0,83 [d, *J* = 12,9 Hz, 2H, CH₂] ; 0,11 [t, *J* = 7,1 Hz, 3H, CH₂(C*H*₃)ₑ] ; -0,56 [t, *J* = 7,1 Hz, 3H, CH₂(C*H*₃)ᵢ] ; -1,91 [d, J = 12,9 Hz, 2H, CH₂] ; -2,04 (s, 2H).
**ESI-HRMS :** calculé m/z = 1387,48063 [M+Na]⁺ ; trouvé 1387,4804.
**UV-VIS** (CH₂Cl₂, λ nm, 10⁻³ ε, M⁻¹.cm⁻¹) : 424 (308,5) ; 518 (17,1) ; 552 (4,0) ; 592 (4,9) ; 648 (1,3).
**FTIR** (KBr, cm⁻¹) : 2240 (ν_{CN}).

Produit obtenu à 36% :
**RMN ¹H (500 MHz, CDCl₃, 300 K) :** δ = 9,06 (s, 4H, β-pyr) ; 8,71 (s, 4H, β-pyr) ; 8,54 (d, *J* = 8,2 Hz, 4H) ; 8,10 (d, *J* = 7,4 Hz, 4H) ; 7,89 (t, *J* = 7,9 Hz, 4H) ; 7,62 (m, 8H) ; 7,08 (s, 4H, NH) ; 6,94 (t, *J* = 7,4 Hz, 4H) ; 6,40 (d, *J* = 7,7 Hz, 4H) ; 3,87 (s, 4H, H_{2'}) ; 2,94 (q, *J* = 7,1 Hz, 4H, CH₂CH₃) ; 1,54 [d, *J* = 12,4 Hz, 4H, CH₂] ; 0,10 (t, *J*= 7,1 Hz, 6H, CH₂*CH*₃) ; -1,41 [d, *J*= 12,4 Hz, 4H, CH₂], -1,96 (s, 2H).

| | | |
|---|---|---|
| **ESI-HRMS :** m/z = | calculé | 1387,48063 [M+Na]⁺ |
| | trouvé | 1387,4784 |

**¹³C NMR (125 MHz, CDCl₃, 300 K) :** 165,6 ; 164,3 ; 138,7 ; 135,9 ; 134,2 ; 133,7; 132,8; 132,3 ; 131,4; 130,0; 129,1 ; 128,6; 128,0; 125,3 ; 124,2; 123,6; 115,3 ; 115,1 ; 61,6; 53,8; 40,2; 12,8.
**UV-VIS** (CH₂Cl₂, λ nm, 10⁻³ ε, M⁻¹.cm⁻¹) : 424 (353,2) ; 518 (18,3) ; 553 (4,0) ; 590 (5,3) ; 647 (1,6).
**FTIR** (KBr, cm⁻¹) : 2240 (ν_{CN}).

### Préparation du composé 19 (voir Figure 9)

### Conditions expérimentales :

Dans un ballon de 250 mL, sous argon, on dissout 215 mg (9,37 mmol) de sodium dans 12 mL d'éthanol absolu. Après 1 heure et 20 minutes d'agitation, on ajoute à l'aide d'une seringue, le cyanoacétate d'éthyle (1,00 mL ; 9,37 mmol). La solution blanchit après quelques minutes. Le mélange est maintenu sous agitation pendant 1 heure. 120 mg (0,0937 mmol) de **17** (obtenu à partir de TAPP αβαβ, selon le protocole utilisé pour préparer **4** comme décrit ci-dessus) sont préalablement dissous dans 30 mL de THF, puis ajoutés goutte à goutte au mélange réactionnel en l'espace de 30 minutes. Le brut est évaporé après 12 heures et lavé avec de l'eau distillée (3 × 50 mL). La phase aqueuse est ensuite précipitée par un mélange de dichlorométhane et de pentane. Le précipité est filtré et redissous dans un minimum de dichlorométhane afin d'être déposé sur colonne de silice. Une montée progressive à 0,2 % en méthanol permet d'obtenir le produit **19** avec un rendement de 60 % (75 mg).
**17 : ¹HNMR (300 MHz, CDCl₃, 323 K) :** δ = 8,95 (s, 8H, β-pyr) ; 8,84 (d, *J* = 8,5 Hz, 4H, aro) ; 8,07 (d, *J*= 8,5 Hz, *J*= 1,2 Hz, 4H, aro) ; 7,92 (t, *J*= 8,5 Hz, *J*= 1,2 Hz, 4H, aro) ; 7,59 (t, *J*= 8,5 Hz, 4H, aro) ; 7,46 (s, 4H, NH) ; 6,77 (d, *J* = 7,5 Hz, 4H, aro) ; 6,65 (d, *J* = 7,5 Hz, 4H, aro) ; 6,57 (t, *J* = 7,5 Hz, 4H, aro) ; 6,30 (s large, 4H, aro) ; 3,35 (s, 8H, CH₂) ; -2,51 (s, NH, 2H).
**19 : ¹HNMR (300 MHz, CDCl₃, 323 K) :** δ = 9,14 (m, 2H, aro) ; 9,08 (s, 4H, β-pyr) ; 8,93 (s, 4H, β-pyr) ; 8,76 (s, 2H, aro) ; 8,07 (d, *J*= 7,3 Hz, 2H, aro) ; 7,93 (m, 8H, aro) ; 7,79 (m, 6H, aro) ; 7,60 (t, *J* = 7,4 Hz, 2H, aro) ; 7,52 (t, *J* = 7,4 Hz, 2H, aro) ; 7,17 (m, 8H, aro + NH) ; 4,94 (s, 2H, H) ; 4,86 (s, 2H, H) ; 2,20 (m, 4H, (CH₂)) ; 1,62 (m, 4H, (CH₂)) ; -0,03 (m, br, 4H, CH₂CH₃ ; -1,46 (t, *J* = 6,9 Hz, 6H, CH₂CH₃) ; -2,09 (s, 2H).

### Métallation de porphyrine par le Bismuth dans l'éthanol

L'intérêt de ce procédé réside dans l'utilisation de l'éthanol absolu. Par ailleurs, la réaction a lieu sous air et à température ambiante.

On dissout 30 mg de la porphyrine base-libre (2,8 × 10⁻⁵ mol) dans 5 mL d'éthanol. On ajoute 30 mg de Bi(NO₃)₃, 5H₂O, (6,2 × 10⁻⁵ mol). La solution se colore instantanément en vert intense. Après 5 minutes d'agitation, on fait buller NH_{3(g)} pendant quelques dizaines de secondes. La solution est évaporée et le mélange chromatographié sur colonne de silice (éluant : 2% MeOH/dichlorométhane). On notera que le résidu se dissout difficilement dans le dichlorométhane. Une fois sur colonne, on observe peu de base libre à séparer. Le produit est enfin précipité dans un mélange dichlorométhane - pentane. Le rendement est quantitatif.

## Revendications

1. Composés répondant à la formule générale (I) suivante : 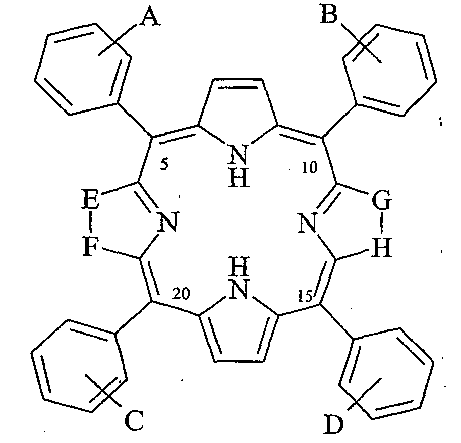 dans laquelle :
- lorsque A forme avec C une chaîne, dite chaîne A-C, de formule (1) suivante :
-X-Y-C₆H₄-(CH₂)ₙ₁-U-(CH₂)ₙ₂-C₆H₄-Y-X- (1)
dans laquelle :
. lorsque X représente NH, O, CO ou CH₂, Y représente respectivement CO, CH₂, NH, ou O,
. n₁ et n₂, indépendamment l'un de l'autre représente un nombre entier compris entre 1 et 3,
. U représente un groupe de la forme C(Z,W) ou N(CHRₐ-COOR_{b}), dans lequel :
. Z représente :
* un groupe électro-attracteur tel que CN, NO₂, ou CO₂⁻,
* ou un groupe CH₂NR₁R₂, dans lequel R₁ et R₂ représentent, indépendamment l'un de l'autre, H, ou un groupe alkyle linéaire, ramifié, ou cyclique, de 1 à 8 atomes de carbone, ou un groupe aryle ou alkylaryle, ou un anticorps déterminé, le cas échéant lié à la partie CH₂N dudit groupe via un espaceur,
* ou un groupement aryle substitué par une fonction SO₃R₃, SO₂R₃, p-NO₂ ou o-NO₂, dans laquelle R₃ représente H, ou un cation choisi parmi les métaux alcalins tels que Na⁺, ou K⁺, ou R₃ représente un groupe NR₄R₅ dans lequel R₄ et R₅ représentent, indépendamment l'un de l'autre, un groupe alkyle linéaire, ramifié, ou cyclique, de 1 à 8 atomes de carbone, ou R₃ représente un groupe para-nitro aryle,
. W représente un groupe CO₂⁻ ou COOR₆ dans lequel R₆ représente H ou un groupe alkyle linéaire, ramifié, ou cyclique, de 1 à 8 atomes de carbone, ou un groupe aryle, ou un alcool appauvri en électrons tel qu'un groupe para-nitro phénol ou ortho-para-nitro phénol,
. ou Z et W forment en association avec l'atome de carbone qui les portent un cycle désigné acide de Meldrum de formule suivante : 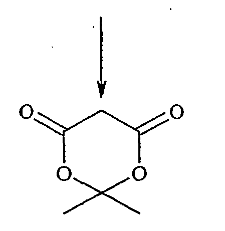
. Rₐ répond à la définition donnée précédemment pour R₁, ou peut également représenter de préférence la chaîne latérale d'un acide aminé, naturel ou modifié,
. R_{b} répond à la définition donnée précédemment pour R₁,
alors B forme avec D une chaîne, dite chaîne B-D, de formule (1) susmentionnée, lesdites chaînes A-C, et B-D, étant situées indépendamment l'une de l'autre, au dessus (position α) ou au dessous (position β) du plan du macrocyle porphyrinique,
- ou lorsque A forme avec D une chaîne, dite chaîne A-D, de formule (1) susmentionnée, alors B forme avec C une chaîne, dite chaîne B-C, de formule (1) susmentionnée, l'une desdites chaînes A-D ou B-C, étant située au dessus (position α) du plan du macrocyle porphyrinique, tandis que l'autre chaîne A-D ou B-C, est située ou au dessous (position β) du plan du macrocyle porphyrinique,
- E représente en association avec F, et H représente en association avec G, indépendamment les uns des autres, CH=CH, ou CH₂-CH₂.

2. Composés selon la revendication 1, **caractérisés en ce que** les enchaînements de formule (1) sont choisis parmi les suivants : 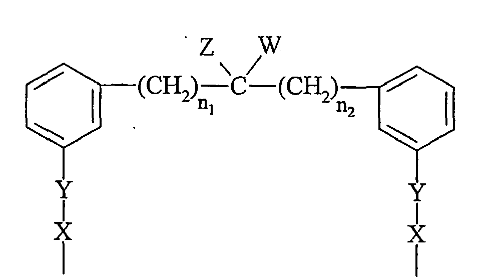 ou 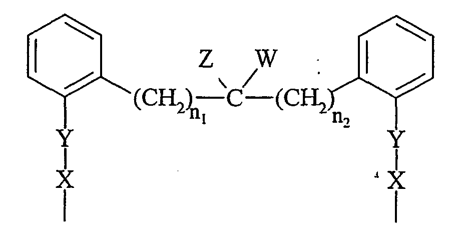 ou 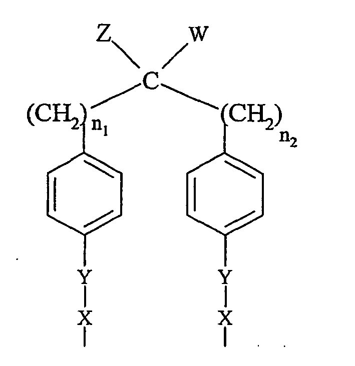 dans lesquels les groupes Z et W sont :
- soit dirigés vers l'intérieur desdits composés et sont situés au dessus ou au dessous du plan du macrocycle porphyrinique suivant que lesdits enchaînements de formule (1) sont situés respectivement en α ou en β, et sont respectivement désignés Ziα et Wiα, ou Ziβ ou Wiβ,
- soit dirigés vers l'extérieur desdits composés, et sont respectivement désignés Ze et We.

3. Composés selon la revendication 1 ou 2, **caractérisés en ce que** A, B, C, et D sont en ortho, et/ou **en ce que** E représente en association avec F, et H représente en association avec G, CH₂-CH₂.

4. Composés selon l'une des revendications 1 à 3, **caractérisés en ce que** A forme avec C, et B forme avec D, des enchaînements de formule (1) respectivement désignés A-C et B-D, ces deux enchaînements étant situés en α.

5. Composés selon la revendication 4, **caractérisés en ce que** :
- les enchaînements A-C et B-D comportent chacun un groupe Ziα et un groupe We,
- ou l'enchaînement A-C comporte un groupe Ziα et un groupe We, tandis que l'enchaînement B-D comporte un groupe Ze et un groupe Wiα,
- ou les enchaînements A-C et B-D comportent chacun un groupe Ze et un groupe Wiα.

6. Composés selon la revendication 4 ou 5, **caractérisés par** les formules suivantes :

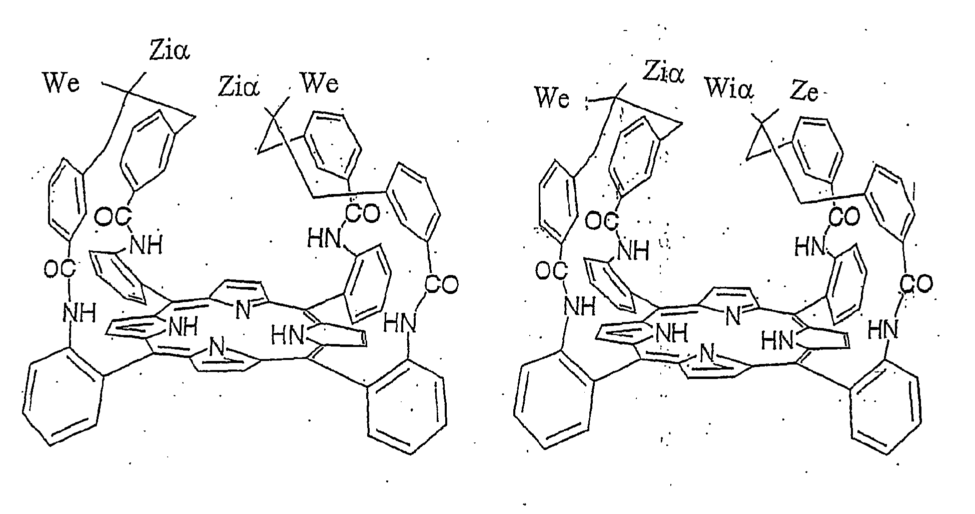

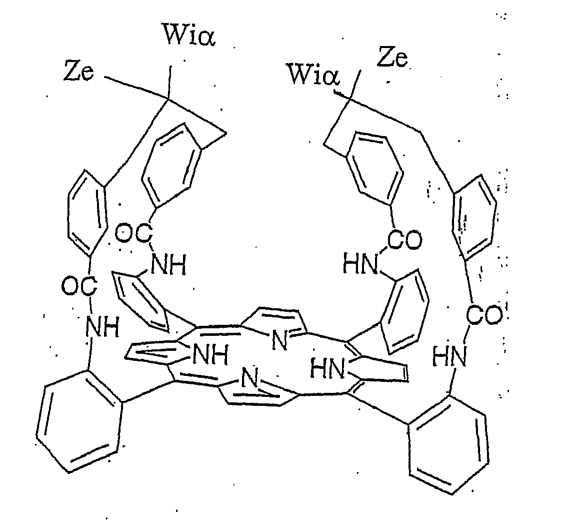

7. Composés selon l'une des revendications 1 à 3, **caractérisés en ce que** A forme avec C un enchaînement A-C de formule (1) situé en position α, et B forme avec D, un enchaînement B-D de formule (1) situé en position β.

8. Composés selon la revendication 7, **caractérisés en ce que** :
- l'enchaînement A-C comporte un groupe Ziα et un groupe We, tandis que l'enchaînement B-D comporte un groupe Ziβ et un groupe We,
- ou l'enchaînement A-C comporte un groupe Ze et un groupe Wiα, tandis que l'enchaînement B-D comporte un groupe Ziβ et un groupe We,
- ou l'enchaînement A-C comporte un groupe Ze et un groupe Wiα, tandis que l'enchaînement B-D comporte un groupe Ze et un groupe Wiβ.

9. Composés selon la revendication 7 ou 8, **caractérisés par** les formules suivantes:

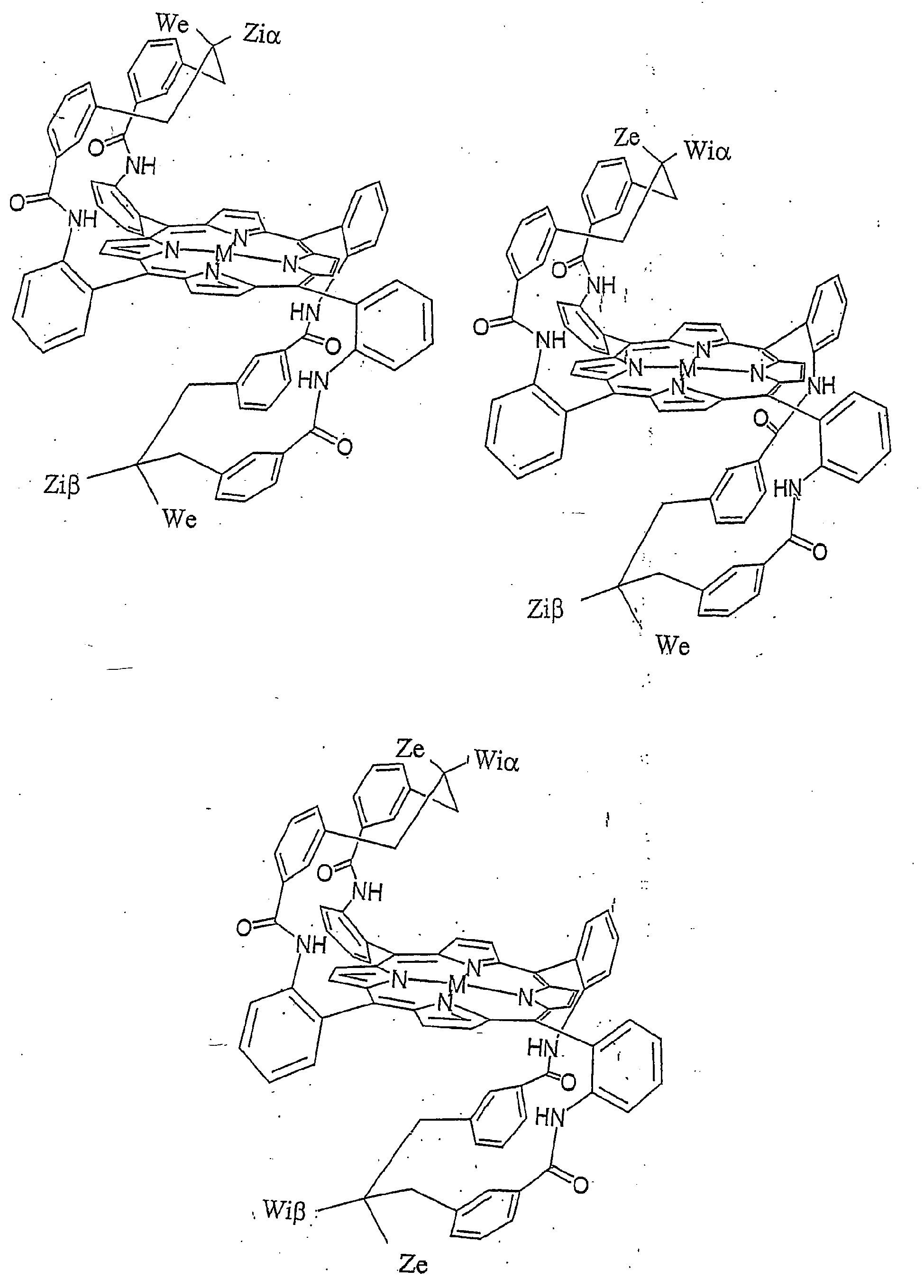

10. Composés selon l'une des revendications 1 à 3, **caractérisés en ce que** A forme avec D un enchaînement A-D de formule (1) situé en position β, et B forme avec C, un enchaînement B-C de formule (1) situé en position α.

11. Composés selon la revendication 10, **caractérisés en ce que** :
- l'enchaînement A-D comporte un groupe Ze et un groupe Wiβ, tandis que l'enchaînement B-C comporte un groupe Ze et un groupe Wiα,
- ou l'enchaînement A-D comporte un groupe Ziβ et un groupe We, tandis que l'enchaînement B-C comporte un groupe Ze et un groupe Wiα,
- ou les enchaînements A-D et B-C comportent chacun un groupe Ziβ et un groupe We.

12. Composés selon la revendication 10 ou 11, **caractérisés par** les formules suivantes :

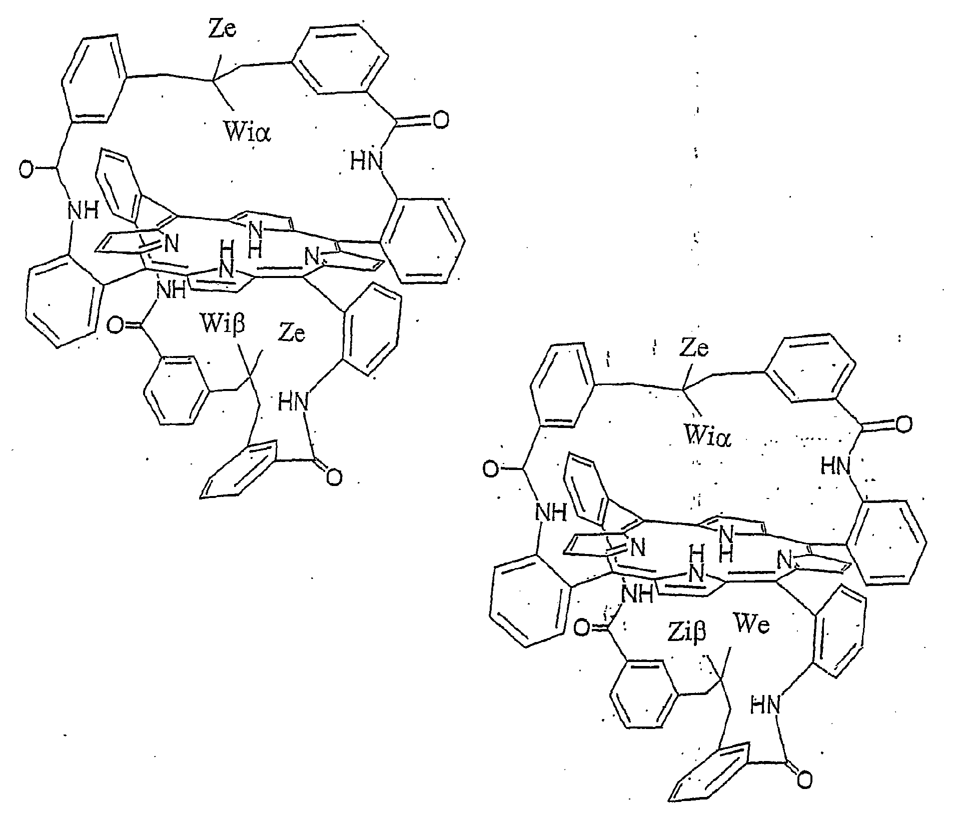

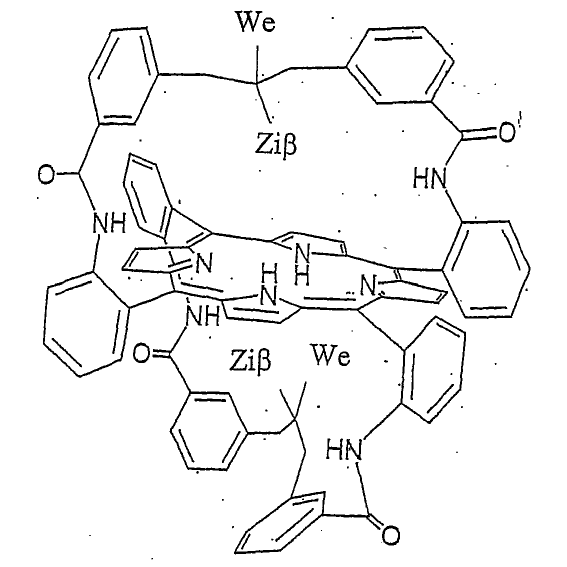

13. Complexes entre un composé selon l'une des revendications 1 à 12, et un radioélément choisi parmi les émetteurs α, ou un élément métallique divalent ou trivalent.

14. Complexes selon la revendication 13 entre un composé selon l'une des revendications 1 à 12, et un radioélément émetteur α choisi parmi le bismuth-212 ou - 213, l'actinium-225, ou l'astate-211.

15. Complexes selon la revendication 13 entre un composé selon l'une des revendications 1 à 12, et un élément métallique divalent ou trivalent choisi parmi Y(III), In(III), Cd(II), Mg(II), Mn(III), Fe(III), B(III) et les lanthanides.

16. Composition pharmaceutique **caractérisée en ce qu'**elle comprend un complexe selon l'une des revendications 13 à 15, en association avec un véhicule pharmaceutiquement acceptable.

17. Composition pharmaceutique selon la revendication 16, **caractérisée en ce qu'**elle se présente sous une forme administrable par voie intraveineuse.

18. Utilisation de complexes définis dans l'une des revendications 13 à 15, pour la préparation d'un médicament destiné au traitement des cancers, ou pour la préparation de compositions destinées à l'imagerie médicale.

19. Utilisation selon la revendication 18 de complexes définis dans l'une des revendications 13 à 15, pour la préparation d'un médicament destiné au traitement des cancers à petites cellules tumorales, tel que la leucémie myéloïde aiguë, les lymphomes non hodgkidiens, les dysplasies bronchopulmonaires, les cancers du sein métastatiques, les cancers colorectaux, les lymphomes, et les pathologies dans lesquelles sont impliqués les motifs antigéniques suivants : CD52, CD22, CD20, HLA-DR, CD33, LE-Y, Ep-CAM, ACE, CAN, EGFR, KSA, VEGF, HER2, GD2, tenascin.

## Claims

1. Compounds corresponding to the following general formula (I): 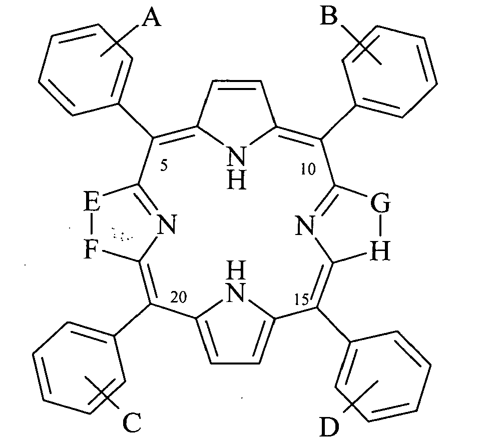 in which:
- when A forms a chain with C, the so-called A-C chain, of formula (1) below:
-X-Y-C₆H₄-(CH₂)ₙ₁-U-(CH₂)ₙ₂-C₆H₄-Y-X- (1)
in which:
. when X represents NH, O, CO or CH₂, Y represents respectively CO, CH₂, NH, or O,
. n₁ and n₂, independently of one another represents an integer comprised between 1 and 3,
. U represents a group of the C(Z,W) or N(CHRₐ-COOR_{b}) form, in which
. Z represents:
* an electroattractive group such as CN, NO₂, or CO₂⁻,
* or a CH₂NR₁R₂ group, in which R₁ and R₂ represent, independently of one another, H, or a linear, branched, or cyclic alkyl group, with 1 to 8 carbon atoms, or an aryl or alkylaryl group, or a specific antibody, if appropriate linked to the CH₂N part of said group via a spacer,
* or an aryl group substituted by an SO₃R₃, SO₂R₃, p-NO₂ or o-NO₂ function, in which R₃ represents H, or a cation chosen from the alkali metals such as Na⁺, or K⁺, or R₃ represents an NR₄R₅ group in which R₄ and R₅ represent, independently of one another, a linear, branched, or cyclic alkyl group, with 1 to 8 carbon atoms, or R₃ represents a para-nitro aryl group,
. W represents a CO₂⁻ or COOR₆ group in which R₆ represents H or a linear, branched, or cyclic alkyl group, with 1 to 8 carbon atoms, or an aryl group, or an alcohol depopulated of electrons such as a para-nitro phenol or ortho-para-nitro phenol group,
. or Z and W form in combination with the carbon atom which carries them a ring designated Meldrum's acid with the following formula: 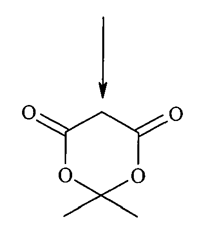
. Rₐ corresponds to the definition previously given for R₁, or can also preferably represent the side chain of a natural or modified amino acid,
. R_{b} corresponds to the definition previously given for R₁,
then B forms a chain with D, the so-called B-D chain, of the abovementioned formula (1), said A-C, and B-D chains, being situated independently of one another, above (α position) or below (β position) the porphyrin macrocycle plane,
- or when A forms a chain with D, the so-called A-D chain, of the abovementioned formula (1), then B forms a chain with C, the so-called B-C chain, of the abovementioned formula (1), one of said A-D or B-C chains being situated above (α position) the porphyrin macrocycle plane, whilst the other A-D or B-C chain, is situated below (β position) the porphyrin macrocycle plane,
- E represents in combination with F, and H represents in combination with G, independently of each other, CH=CH, or CH₂-CH₂.

2. Compounds according to claim 1, **characterized in that** the chain formations of formula (1) are chosen from the following: 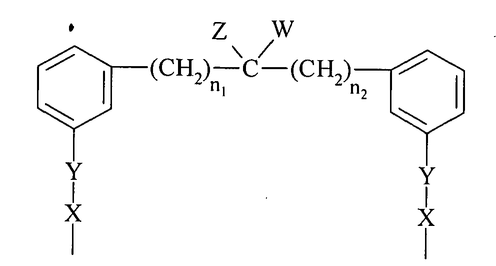 or 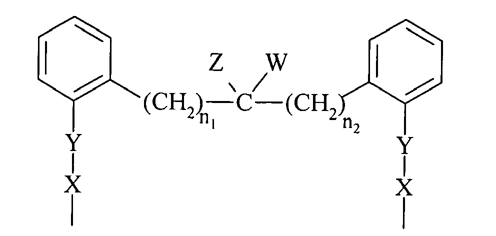 or 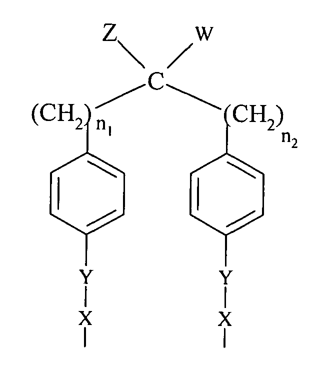 in which the Z and W groups are:
- either directed towards the interior of said compounds and are situated above or below the porphyrin macrocycle plane according to whether said chain formations of formula (1) are situated respectively in α position or in β position, and are respectively designated Ziα and Wiα, or Ziβ or Wiβ,
- or directed towards the exterior of said compounds, and are respectively designated Ze and We.

3. Compounds according to claim 1 or 2, **characterized in that** A, B, C, and D are in ortho position, and/or **in that** E represents in combination with F, and H represents in combination with G, CH₂-CH₂.

4. Compounds according to one of claims 1 to 3, **characterized in that** A forms with C, and B forms with D, chain formations of formula (1) respectively designated A-C and B-D, these two chain formations being situated in α position.

5. Compounds according to claim 4, **characterized in that**:
- the A-C and B-D chain formations each comprise a Ziα group and a We group,
- or the A-C chain formation comprises a Ziα group and a We group, whilst the B-D chain formation comprises a Ze group and a Wiα group,
- or the A-C and B-D chain formations each comprise a Ze group and a Wiα group.

6. Compounds according to claim 4 or 5, **characterized by** the following formulae:

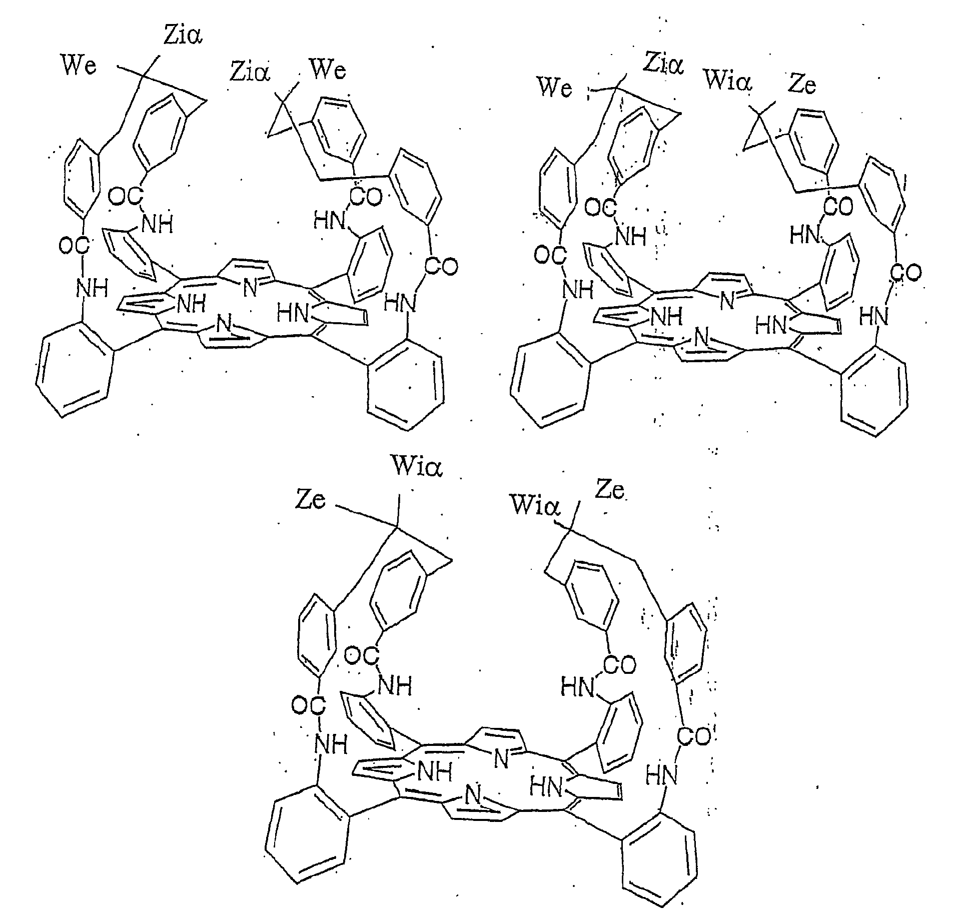

7. Compounds according to one of claims 1 to 3, **characterized in that** A forms with C an A-C chain formation of formula (1) situated in α position, and B forms with D, a B-D chain formation of formula (1) situated in β position.

8. Compounds according to claim 7, **characterized in that**:
- the A-C chain formation comprises a Ziα group and a We group, whilst the B-D chain formation comprises a Ziβ group and a We group,
- or the A-C chain formation comprises a Ze group and a Wiα group, whilst the B-D chain formation comprises a Ziβ group and a We group,
- or the A-C chain formation comprises a Ze group and a Wiα group, whilst the B-D chain formation comprises a Ze group and a Wiβ group.

9. Compounds according to claim 7 or 8, **characterized by** the following formulae:

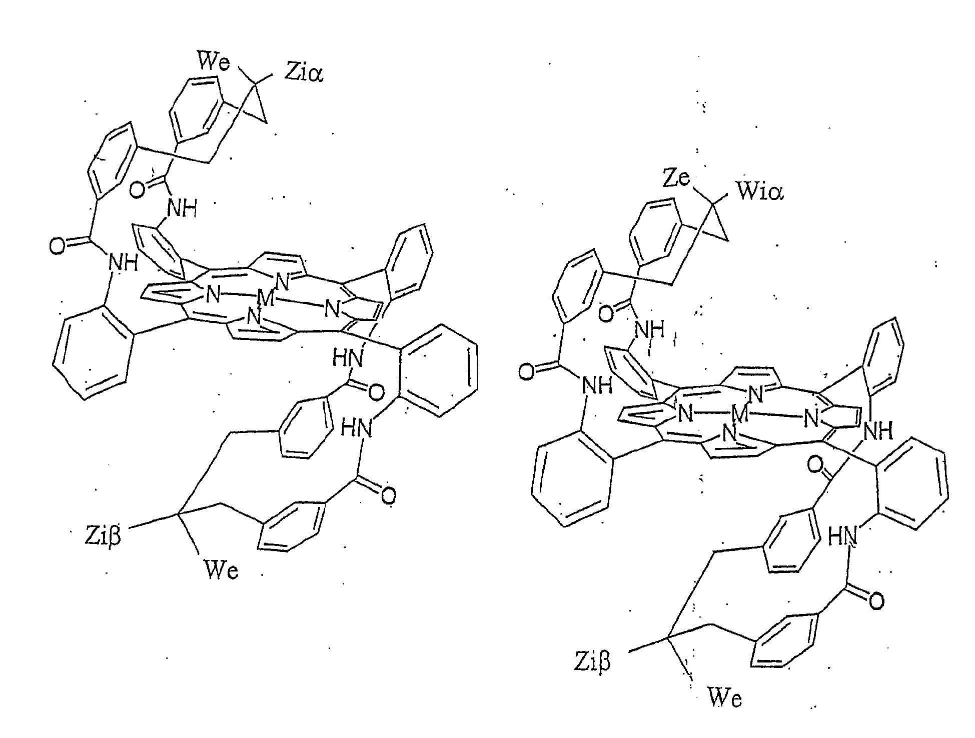

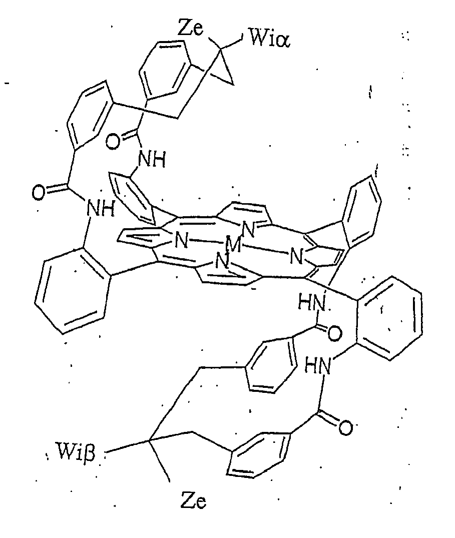

10. Compounds according to one of claims 1 to 3, **characterized in that** A forms with D an A-D chain formation of formula (1) situated in β position, and B forms with C, a B-C chain formation of formula (1) situated in α position.

11. Compounds according to claim 10, **characterized in that**:
- the A-D chain formation comprises a Ze group and a Wiβ group, whilst the B-C chain formation comprises a Ze group and a Wiα group,
- or the A-D chain formation comprises a Ziβ group and a We group, whilst the B-C chain formation comprises a Ze group and a Wiα group,
- or the A-D and B-C chain formations each comprise a Ziβ group and a We group.

12. Compounds according to claim 10 or 11, **characterized by** the following formulae:

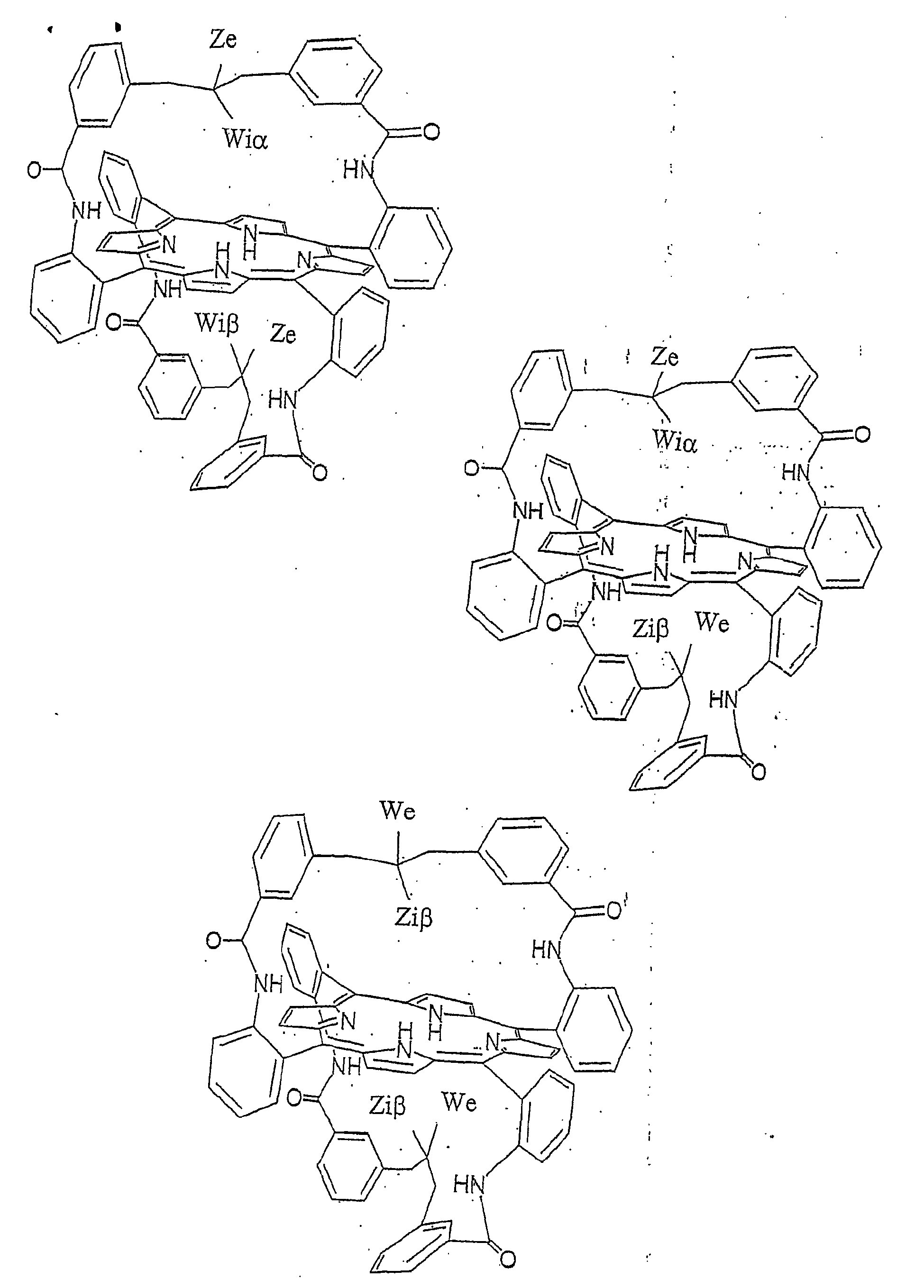

13. Complexes between a compound according to one of claims 1 to 12, and a radioelement chosen from the α emitters, or a divalent or trivalent metallic element.

14. Complexes according to claim 13 between a compound according to one of claims 1 to 12, and an α-emitter radioelement chosen from bismuth-212 or -213, actinium-225, or astatine-211.

15. Complexes according to claim 13 between a compound according to one of claims 1 to 12, and a divalent or trivalent metallic element chosen from Y(III), In(III), Cd(II), Mg(II), Mn(III), Fe(III), B(III) and the lanthanides.

16. Pharmaceutical composition **characterized in that** it comprises a complex according to one of claims 13 to 15, in combination with a pharmaceutically acceptable vehicle.

17. Pharmaceutical composition according to claim 16, **characterized in that** it is presented in a form which can be administered by intravenous route.

18. Use of complexes defined in one of claims 13 to 15, for preparing a medicament intended for the treatment of cancer, or for preparing compositions intended for medical imaging.

19. Use according to claim 18 of complexes defined in one of claims 13 to 15, for preparing a medicament intended for the treatment of tumorous small-cell cancers, such as acute myeloid leukemia, non-Hodgkin's lymphomas, bronchopulmonary dysplasias, metastatic breast cancers, colorectal cancers, lymphomas, and pathologies in which the following antigenic units are involved: CD52, CD22, CD20, HLA-DR, CD33, LE-Y, Ep-CAM, ACE, CAN, EGFR, KSA, VEGF, HER2, GD2, tenascin.

## Patentansprüche

1. Verbindungen nach der folgenden allgemeinen Formel (I) : 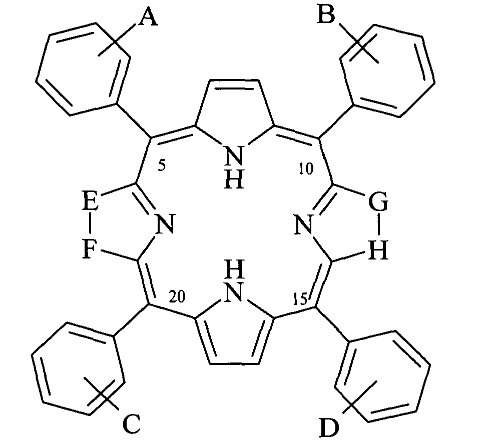 worin :
- wenn A mit C eine Kette oder A-C Kette bildet, mit der folgenden Formel (1) :
-X-Y-C₆H₄-(CH₂)ₙ₁-U-(CH₂)ₙ₂-C₆H₄-Y-X- (1)
worin :
. wenn X NH, O, CO oder CH₂ bedeutet, Y jeweils CO, CH₂, NH, oder O bedeutet,
. n₁ und n₂ unabhängig eine ganze Zahl zwischen 1 und 3 bedeuten,
. U eine Gruppe der Formel C(Z,W) oder N(CHRₐ-COOR_{b}) bedeutet, worin :
. Z :
* eine elektroanziehende Gruppe, wie CN, NO₂ oder CO₂⁻,
* oder eine CH₂NR₁R₂ Gruppe, worin R₁ und R₂ unabhängig H, oder eine lineare, verzweigerte oder zyklische Gruppe mit 1 bis 8 Kohlenstoffatomen, oder eine aryl- oder alkylaryl- Gruppe, oder einen vorherbestimmten Antikörper, der gegebenenfalls durch einen Spacer mit dem CH₂N Teil der genannten Gruppe gebindet wird, bedeuten,
* oder eine Aryl Gruppe, die mit einer SO₃R₃, SO₂R₃, p-NO₂ oder o-NO₂ Funktion substituiert ist, worin R₃ H, oder ein Kation, das unter alkalinischen Metallen wie Na⁺ oder K⁺ gewählt wird, bedeutet, oder R₃ eine NR₄R₅ Gruppe bedeutet, worin R₄ und R₅ unabhängig eine C1 - C8 lineare, verzweigte oder zyklische Gruppe bedeuten, oder R₃ eine Para-nitro aryl-Gruppe bedeutet, bedeutet,
. W eine CO₂⁻ oder COOR₆ Gruppe bedeutet, worin R₆ H oder eine C1 - C8 lineare, verzweigte oder zyklische Gruppe, oder eine Arylgruppe, oder ein Alkohol, der erlektronenverarmt ist, wie eine Para-nitro phenol- oder eine ortho-para-nitro phenol- Gruppe, bedeutet,
. oder Z und W mit dem sie tragenden Kohlenstoffatom einen Zyklus, d.h. eine sogenannte Meldrum-Säure mit der folgenden Formel bilden : 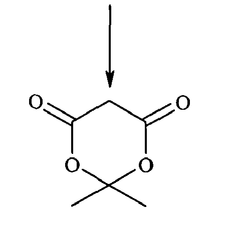
. Rₐ folgt der obengegebenen Definition für R₁, oder kann auch vorzugsweise die Seitenkette einer natürlichen oder modifizierten Aminosäure bedeuten,
. R_{b} folgt der obengegebenen Definition für R₁,
dann B mit D eine Kette bildet, und zwar die sogenannte B-D Kette, mit der obengenannten Formel (1), worin die obengenannten A-C und B-D Ketten unabhängig voneinander oben (α-Stellung) oder unten (β-Stellung) im Verhältnis zur Ebene des Porphyrin-Makrozyklus liegen,
- oder wenn A mit D eine Kette bildet, die sogenannte A-D Kette, mit der obengenannten Formel (1), dann B mit C eine Kette bildet, die sogenannte B-C Kette, mit der oben genannten Formel (1), worin eine der obengenannten A-D oder B-C Ketten oben (α-Stellung) im Verhältnis zur Ebene des Porphyrin-Makrozyklus liegt, während die andere A-D oder B-C Kette unten (β-Stellung) im Verhältnis zur Ebene des Porphyrin-Makrozyklus liegt,
- E zusammen mit F, und H zusammen mit G, unabhängig voneinander für CH=CH oder CH₂-CH₂ stehen.

2. Verbindungen nach Anspruch 1, **dadurch gekennzeichnet, daß** die Formel (1) Ketten unter den folgenden gewählt sind : 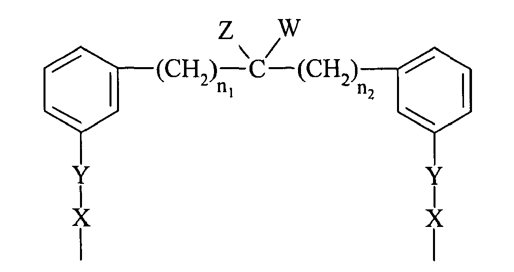 oder 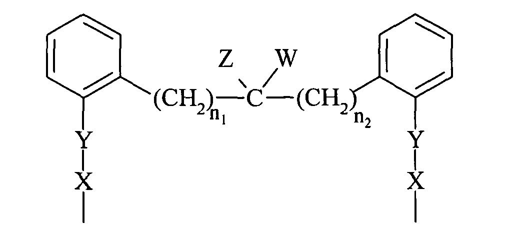 oder 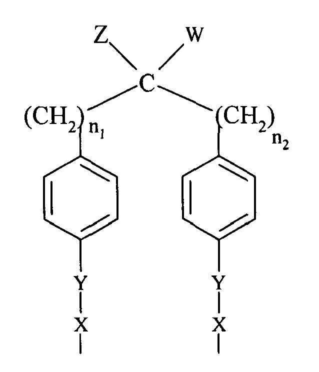 worin die Z und W Gruppen :
- entweder zum Inneren der obengenannten Verbindungen, und entweder oben oder unten im Verhältnis zur Ebene des Prophyrin-Makrozyklus liegen, je nachdem ob diese Formel (1) Ketten jeweils in der α- oder β-Stellung liegen, und jeweils Ziα und Wiα, oder Ziβ oder Wiβ genannt sind,
- oder zum Außeren der obengenannten Verbindungen orientiert, und jeweils Ze und We genannt sind.

3. Verbindungen nach Anspruch 1 oder 2, **dadurch gekennzeichnet, daß** A, B, C, und D in ortho Stellung sind, und/oder daß E mit F, und H mit G, CH₂-CH₂ bedeuten.

4. Verbindungen nach irgendwelcher Anspruch 1 bis 3, **dadurch gekennzeichnet, daß** A mit C, und B mit D, Formel (1) Ketten bildet, die jeweils A-C und B-D genannt werden, worin diese zwei Ketten in α Stellung sind.

5. Verbindungen nach Anspruch 4, **dadurch gekennzeichnet, daß** :
- jede A-C und B-D Kette eine Ziα- und eine We-Gruppe beinhaltet,
- oder die A-C Kette eine Ziα- und eine We-Gruppe beinhaltet, während die B-D Kette eine Ze- und eine Wiα-Gruppe beinhaltet,
- oder die A-C und B-D Ketten jede eine Ze-Gruppe und eine Wiα-Gruppe beinhalten.

6. Verbindungen nach Anspruch 4 oder 5, die durch die folgenden Formeln **gekennzeichnet** sind :

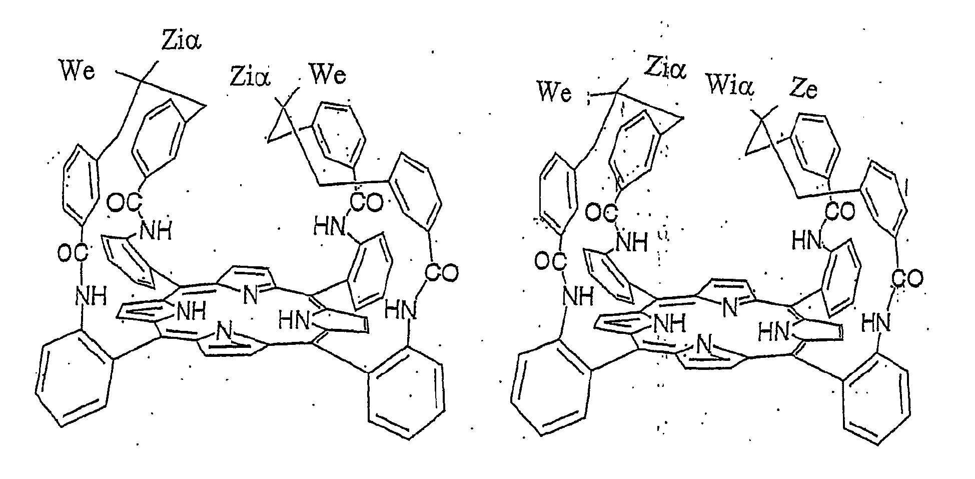

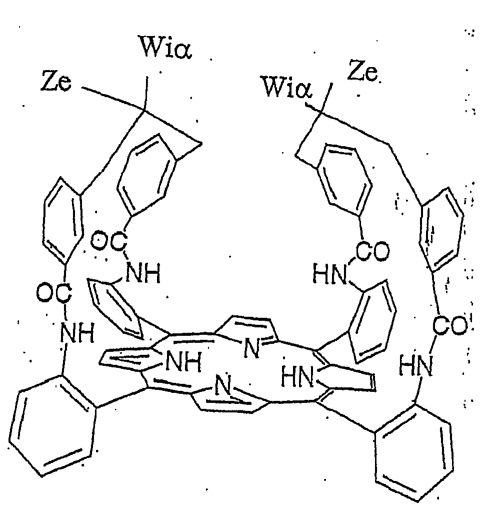

7. Verbindungen nach irgendwelcher Anspruch 1 - 3, **dadurch gekennzeichnet, daß** A mit C eine A-C Kette der Formel (1), die in α-Stellung liegt, und daß B mit D eine B-D Kette der Formel (1), die in β-Stellung liegt.

8. Verbindungen nach Anspruch 7, **dadurch gekennzeichnet, daß** :
- die A-C Kette eine Ziα- und eine We-Gruppe beinhaltet, während die B-D Gruppe eine Ziβ - und eine We-Gruppe beinhaltet,
- oder die A-C Kette eine Ze- und eine Wiα-Gruppe beinhaltet, während die B-D Kette eine Ziβ- und eine We-Gruppe beinhaltet.
- oder die A-C Kette eine Ze- und eine Wiα-Gruppe beinhaltet, während die B-D Kette eine Ze- und eine Wiβ-Gruppe beinhaltet.

9. Verbindungen nach Anspruch 7 oder 8, die durch die folgenden Formeln **gekennzeichnet** sind :

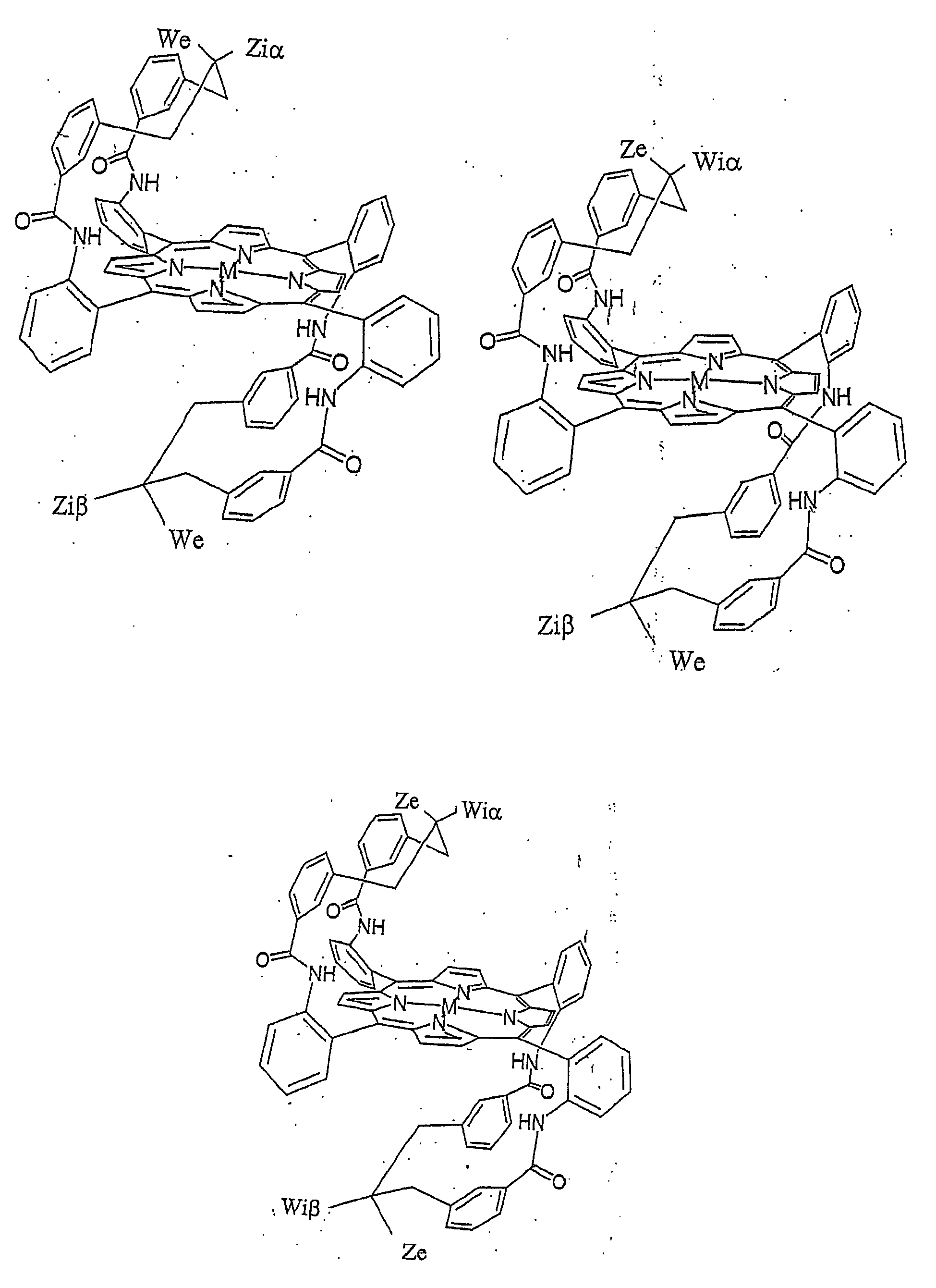

10. Verbindungen nach irgendwelcher Anspruch 1 - 3, **dadurch gekennzeichnet, daß** A mit D eine A-D Kette der Formel (1) bildet, das in β-Stellung liegt, und daß B mit C eine B-C Kette der Formel (1) bildet, das in position α-Stellung liegt.

11. Verbindungen nach Anspruch 10, **dadurch gekennzeichnet, daß** :
- die A-D Kette eine Ze-Gruppe und eine Wiβ-Gruppe beinhaltet, während die B-C Kette eine Ze-Gruppe und eine Wiα-Gruppe beinhaltet,
- oder die A-D Kette eine Ziβ-Gruppe und eine We-Gruppe beinhaltet, während die B-C Kette eine Ze-Gruppe und eine Wiα-Gruppe beinhaltet,
- oder die A-D und B-C Ketten jede eine Ziβ-Gruppe und eine We-Gruppe beinhalten.

12. Verbindungen nach Anspruch 10 oder 11, die mit den folgenden Formeln **gekennzeichnet** sind :

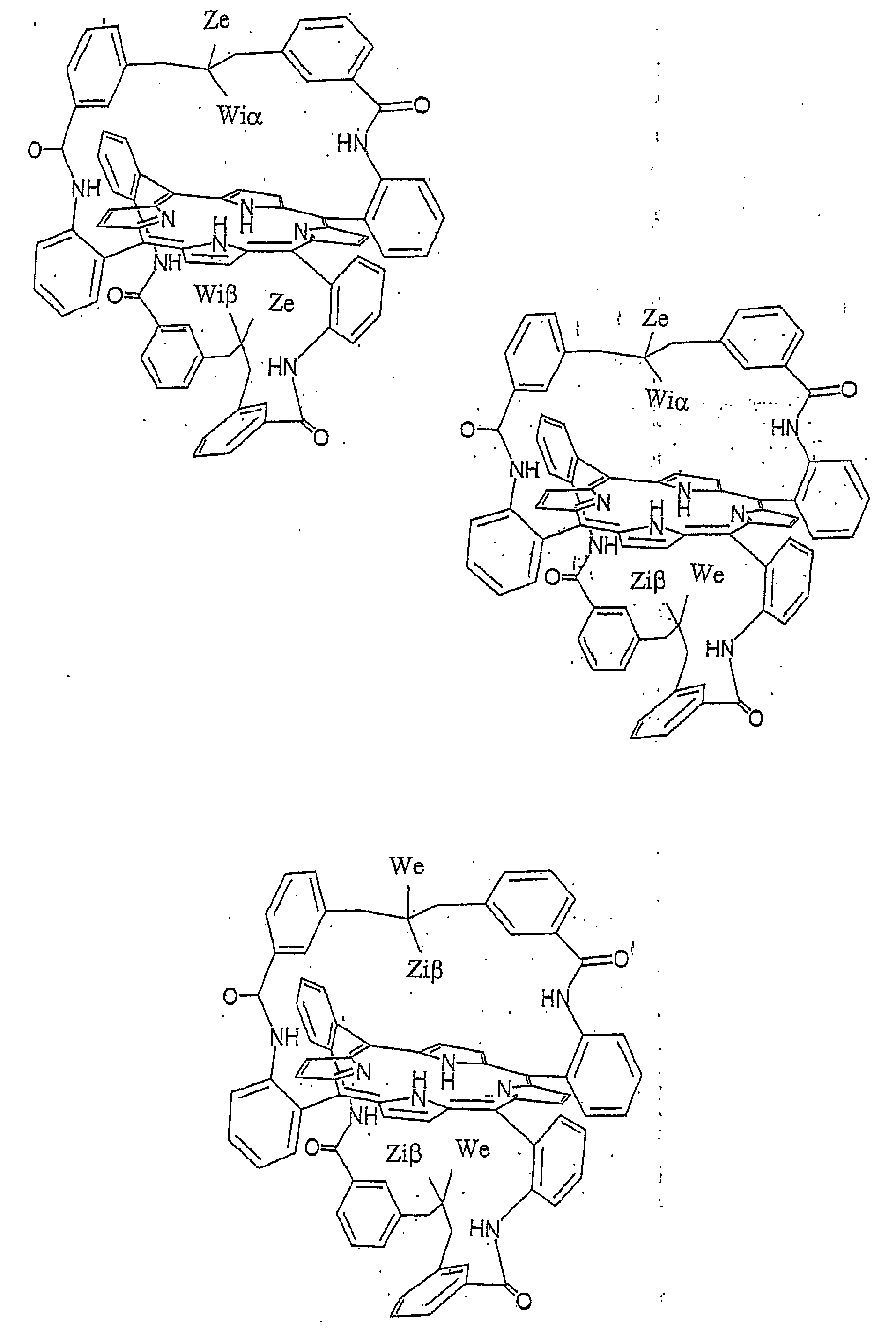

13. Komplexe zwischen einer Verbindung nach irgendwelcher Anspruch 1 - 12 und einem radioaktiven Element, das zwischen den α-Sendern oder einem zweiwertigen oder dreiwertigen Element gewählt wird.

14. Komplexe nach Anspruch 13 zwischen einer Verbindung nach irgendwelcher Anspruch 1 - 12 und einem Radiosender Element α, das zwischen Wismut-212 oder - 213, Actinium-225 oder Astatin-211 gewählt ist.

15. Komplexe nach Anspruch 13 zwischen einer Verbindung nach irgendwelcher Anspruch 1 - 12 und einem zwei- oder dreiwertigen Metallelement, das zwischen Y(III), In(III), Cd(II), Mg(II), Mn(III), Fe(III), B(III) und Lanthaniden gewählt ist.

16. Pharmazeutische Zusammenstellung, **dadurch gekennzeichnet, daß** sie einen Komplex nach irgendwelcher Anspruch 13 - 15 mit einem pharmazeutischen annehmbaren Träger beinhaltet.

17. Pharmazeutische Zusammenstellung nach Anspruch 16, **dadurch gekennzeichnet, daß** sie unter einer intravenös verabreichbaren Form vorliegt.

18. Verwendung von Komplexen wie in irgendwelcher Anspruch 13 - 15 definiert, für die Herstellung eines Arzneimittel für die Behandlung von Krebs, oder für die Herstellung von Zusammensetzungen, die für das Radioimaging geignet sind.

19. Verwendung nach Anspruch 18 von Komplexen wie in irgendwelcher Anspruch 13 - 15 definiert, für die Herstellung eines Arzneimittels für die Behandlung von kleinen Tumorzellen-Krebsen, wie akute myeloische Leukose, nicht lymphogranulomatöse Lymphome, bronchopulmonale Dysplasien, metastatische Brustkrebse, kolorektale Krebse, Lymphome und Pathologien mit den folgenden antigen Motiven : CD52, CD22, CD20, HLA-DR, CD33, LE-Y, Ep-CAM, ACE, CAN, EGFR, KSA, VEGF, HER2, GD2, Tenascin.
